# EUROPEAN PATENT SPECIFICATION

(11) **EP 2 131 769 B1**
(45) Date of publication and mention of the grant of the patent: **27.04.2011**
(21) Application number: 08731298.9
(22) Date of filing: 03.03.2008
(51) Int. Cl.: A61B 17/88, A61B 17/72, A61B 17/70

(54) **FRACTURE FIXATION SYSTEM**
FRAKTUR-FIXATIONSSYSTEM
SYSTÈME DE RÉPARATION DE FRACTURE

(30) Priority: 02.03.2007 US 904578 P
(43) Date of publication of application: 16.12.2009
(73) Proprietor: Spinealign Medical, Inc., San Jose, CA 95112 (US)
(72) Inventor: CHIRICO, Paul, E., Campbell, CA 95005 (US); CHAN, Benny, M., Fremont, CA 94555 (US); PAKBAZ, R., Sean, San Jose, CA 95112 (US); HORTON, Joseph, A., San Jose, CA 95112 (US); SOUZA, Allison, Santa Clara CA 95050 (US); MARTINI, Brian, Aptos, CA 95003 (US)
(74) Representative: Pugh, Robert Ian
(86) International application number: PCT/US2008/055723
(87) International publication number: WO 2008/109566

(56) References cited:
- EP-A- 1 582 159
- WO-A-97/18769
- FR-A- 2 653 006
- US-A1- 2002 165 544
- US-A1- 2006 084 998
- US-A1- 2006 184 192
- US-A1- 2007 067 034
- US-B1- 6 332 885

## Description

### CROSS REFERENCE TO RELATED APPLICATIONS

This application claims priority to U.S. Provisional Patent Application No. 60/904,578 of Chirico et al., (US 2009005783) entitled "Fracture Fixation System and Method", filed on March 2, 2007.

### FIELD OF THE INVENTION

The invention relates to a system and methods of using the system to securely fix aligned bone fracture segments in place to promote optimal healing of the fracture.

### INCORPORATION BY REFERENCE

All publications and patent applications mentioned in this specification are herein incorporated by reference in their entirety to the same extent as if each individual publication or patent application was specifically and individually indicated to be incorporated by reference.

### BACKGROUND OF THE INVENTION

The goal of bone fracture fixation is to stabilize bone regions around the fracture in an optimal alignment, and by such stabilized and supported alignment, allow fast healing of the fracture, and a return to mobility and function of the fracture and surrounding region as a whole. Fracture fixation methods are generally categorized as external or internal. Internal fixation methods are more interventional and surgical in nature than external fixation methods, and they may also be complemented by the support of external methods. External fixation typically includes a closed reduction to restore or maintain alignment of fractured regions, which is then stabilized by splints, casts, and slings. External traction can also be applied to the fracture, taking advantage of leverage that can be applied to these external structures. Internal fixation methods include the interventional use of various hardware elements such as wires, pins and screws, plates, intramedullary nails or rods, staples, and clamps. Internal fixation devices and approaches have also created an avenue for introducing bioactive agents into the fracture site, such osteoinductive agents or anti-infective agents, that can encourage bone healing and combat infections. Bone fractures are by their nature highly individual and complex. It would therefore be beneficial to provide new devices and methods, particularly those that can readily be tailored to fracture specifics and create minimal collateral disturbance.

US 2002/165544 describes an intramedullary nail comprising a distal radially expandable section, a proximal radially expandable section and a non-expandable middle section.

US-B-6,332,885 discloses a device comprising an alloy shape-memory cylinder having a middle region and two end regions which are split outwardly.

US 2006/084998 describes a device comprising a tubular body and a plurality of spline elements extending from the distal and proximal ends of the tubular body. The splines can be moved from a collapsed state to an expanded state.

US 2006/184192 discloses a system for creating a cavity in an interior body. The system comprised a shaft having a proximal portion, a distal portion and an expandable portion therebetween.

FR-A-2653006 describes an intramedullary nail comprising a tubular body comprising a threaded rod and at least two expansion means which are engaged respectively on two threads of the threaded rod.

WO 97/18769 discloses an intramedullary nail having expandable sleeves (3, 4) slidably mounted on an axial rod. Tightening a control nut axially compresses tubular spacers, thereby causing expansion of the expandable sleeves.

EP-A-1582159 describes an intramedullary nail comprising a substantially straight stem and at least one expansion means situated at the distal and/or proximal ends of the stem.

### SUMMARY OF THE INVENTION

The invention provided herein relates to a system for stabilizing a bone fracture as defined in claim 1. Preferred embodiments are set forth in the dependent claims. Methods, not claimed, for applying that system are also described. The system includes a first anchorable member and a second anchorable member, each member having central passageway, each member having a constrained non-anchoring configuration and a released anchoring configuration. The system further includes a connector having a central passageway, the connector configured, to be attached to the proximal end of the first anchorable member and the distal end of the second anchorable member, such that the central passageways of the anchorable members and the connector form a continuous passageway. The system may further include delivery devices, devices that rotate or otherwise manipulate the system in situ, and devices for injection of bone-filling compositions. The first anchorable member, the second anchorable member and the connector may be collectively referred to as a fracture-stabilizing device.

The first and second anchorable members and the connector are all separate elements. The anchorable members and the connector may be assembled during the delivery and anchoring of the device to the target fracture site.

In typical embodiments, the constrained (*e.g*., non-anchoring) configuration of an anchorable member is substantially linear in form, and the released (*e.g*., anchoring) configuration includes a radially expanded structure. In some variations, the non-anchoring configuration of the member includes three or more flat surfaces in cross section; some of these embodiments may have a rectangular cross section. In other embodiments, the member has a rounded configuration in the unexpanded state. In some embodiments, the released configuration with a radially expanded structure includes expandable struts. In various embodiments of the struts, they may present a flat or a rounded surface as a leading edge. More preferably, the struts of the self-expanding members may include a cutting edge that is sharp and sufficiently strong to cut into bone. This leading edge may be a knife-edge, a serrated edge, or the like. In some embodiments, the expandable struts form a symmetrical bow when freely expanded; in other embodiments they may form an asymmetrical bow. In strut embodiments that form an asymmetrical bow, the asymmetry may include a bow that has its greatest radial diameter distributed either distally or proximally.

The passageways of the first anchorable member, the connector, and the second anchorable member may be adapted to convey a flowable material such as a bone filling composition or cement, which may include biological materials, synthetic materials, inorganic materials, or bioactive agents (or any combinations thereof). The connector may include holes for egress of the flowable material. In some embodiments, the passageway may include a hollow tube extending through the anchorable members and/or the connector, and the hollow tube may also contain holes for egress of flowable material, or may be rupturable to release flowable material.

The system may also include a delivery device for delivering the fracture-stabilization device into the bone in the collapsed (unexpanded) state and for delivery or release of the device within the fracture region and attachment. Because the fracture-stabilization device is at least partially self-expanding, and may be biased into an expanded (anchoring) state, the delivery device may apply force to maintain the fracture-stabilization device in a delivery (collapsed) configuration. For example, a delivery device may include one or more rods. These rods may be configured to releasably engage one or both of the anchorable members. For example, the distal anchorable member may include an attachment site at its distal end configured to releasably attach to a delivery device. In some variations the other (proximal) member includes a second attachment site that can be releasable attached to another portion of the delivery device. The delivery device may therefore apply force to keep the fracture-stabilization device in the collapsed (delivery) configuration. In variations in which the components expandable members and/or connector of the fracture-stabilization device are delivered separately, each component portion may include attachment sites at either end to maintain the delivery configuration.

The connector and at least one of the first or second anchorable members may be threadably connected such that rotation of one of the anchorable members (or the connector) changes the distance between the two anchorable members. Thus, the relative spacing of the members may be adjusted (*e.g*., by rotating). In some variations, the connector is adapted to modify the length between the anchoring members. The spacing may be increased or decreased. The spacing may be modified either during implantation (in the contracted state) or after implantation (in the expanded state).

In general, the delivery device may be configured to position the first anchorable member, the connector, and the second anchorable member into a bone fracture site. The delivery device may be configured to releasably attach to one end of first anchorable member. In some embodiments that include a delivery device, the device includes a rod that is configured to engage the fracture-stabilization device (or a component of the device) at some location distally from the first end. For example, the rod may extend distally from the delivery device into the continuous passageway, and the rod may be configured to attach to the distal end of the first anchorable member, an end of the connector, or either end of a second member. The delivery device may separately apply force to maintain each expandable member in a collapsed configuration. For example, parallel or telescoping rods may extend in the central passage and attach to various components to apply force sufficient to keep individual members in the collapsed (delivery) configuration or to provide force to expand either or both members of the fracture-stabilization device.

A delivery device for a fracture-stabilization system may also include a sleeve or cannula that encloses (*e.g*., at least partially radially surrounds) the first anchorable member, the connector, and the second anchorable member. A sleeved delivery device may also include a push (or push/pull) rod configured to extend distally from the applicator to the proximal end of the second anchorable member.

Any of the delivery devices described herein may also be configured to allow removal or readjustment of the fracture-stabilization devices. For example, the connectors between the fracture-stabilization device and the delivery device (*e.g*., rods, sleeves, etc.) may be reengaged so that the device can be partially collapsed and adjusted or removed.

Also described herein are methods for stabilizing a fractured bone using a fracture-stabilization device. For example, a method of stabilizing a fracture bone may include: forming a passage in the fractured bone through a proximal bone region, across the fracture, and into a distal bone region; positioning a bone fracture-stabilizing system having a fust expandable member a connector and a second expandable member in the passage; and anchoring the first anchoring member within the distal bone region and the second anchoring member within the proximal bone region. The method may begin with aligning the proximal bone region and the distal bone region prior to forming the passage in the bone.

The method may also include inserting the anchorable members of the fracture-stabilization device into the passage in a constrained configuration. The anchoring members may be released from the constrained configuration to expand and anchor. For example a fracture-stabilization device may be anchored by detaching the first anchoring member from a rod of the delivery device.

The method may include radially expanding a plurality of bowed struts from each anchorable member to anchor the member within the bone. The struts radially self-expand. Struts may be expanded with a mechanical assist after self-expanding. The first and second anchorable members may expand simultaneously. Alternatively, the first anchorable member expands before the second anchorable member, or vice-versa.

The method may also include cutting the bone as the device expands. For example, the method may include the step of exposing cutting surfaces on the struts as they expand into the anchoring configuration.

The method may also include applying a flowable material through the continuous passageway. The flowable material may exit the passageway into the surrounding bone. For example, the flowable material may exit openings in the passageway of the first and second member, and/or the connector, flowing a material through the continuous passageway so that at least some material exits holes from the connector. The flowable material may be hardened (*e*.*g*. by setting, curing, or otherwise) to form a solid material.

The method of stabilizing a bone fracture may also include the step of altering the distance between the first and second anchoring members. For example, the connector may be rotated to change the distance between the first and second anchoring members.

### BRIEF DESCRIPTION OF THE FIGURES

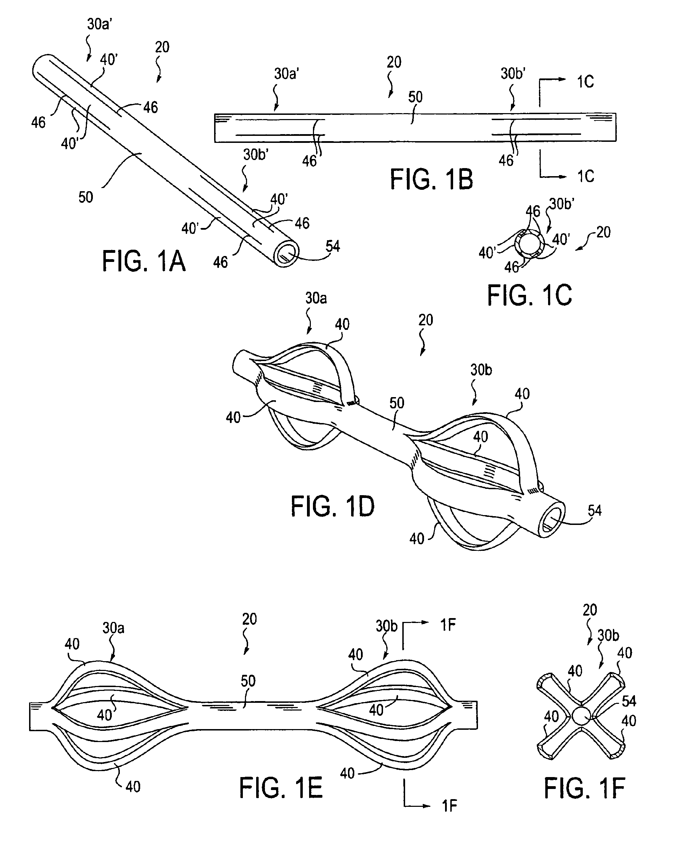

Figures 1A-1F, 2A-2F, 3A-3F, 4A-4F, 5A-5F, 6-8, 9A, 9B, 10A-10F, 11A-11D, 12A-12E, 13 and 14 show embodiments which are not claimed:

**FIGS. 1A -1F** show a fracture-stabilization device with a circular cross-section having two expandable members, each with four radially expandable struts. The struts have a flat expanding surface. **FIG. 1A** is a perspective view of the body of the device. **FIG. 1B** is a side view of the body of the device showing slots forming the struts. **FIG. 1C** is a cross-sectional view of the device. **FIG. 1D** is a perspective view of the device after the struts have radially expanded. **FIG. 1E** is a side view of the device after the struts have radially expanded. **FIG. 1F** is an end view of the device after the struts have radially expanded.

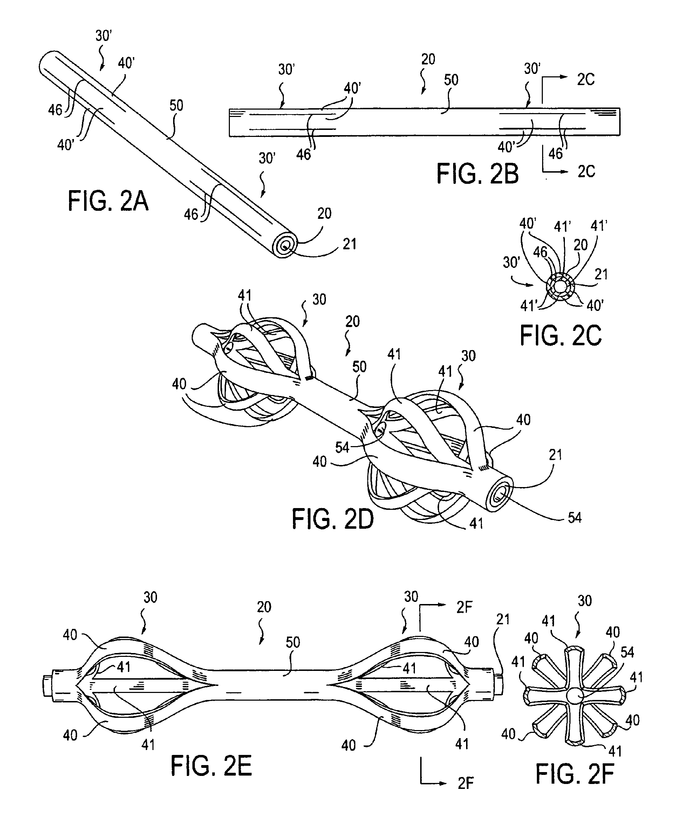

**FIGS. 2A -2F** show an internal-external, or double-bodied, fracture-stabilization device, wherein each body includes two expandable members (or regions), each with four expandable struts. The struts of the internal and external bodies are staggered with respect to each other. **FIG. 2A** is a perspective view of the device in the unexpanded (insertion) configuration. **FIG. 2B** is a side view of the body of the device. **FIG. 2C** is a cross-sectional view of the device. **FIG. 2D** is a perspective view of the device after the struts have radially expanded. **FIG. 2E** is a side view of the device after the struts have radially expanded. **FIG. 2F** is an end view of the device after the struts have radially expanded.

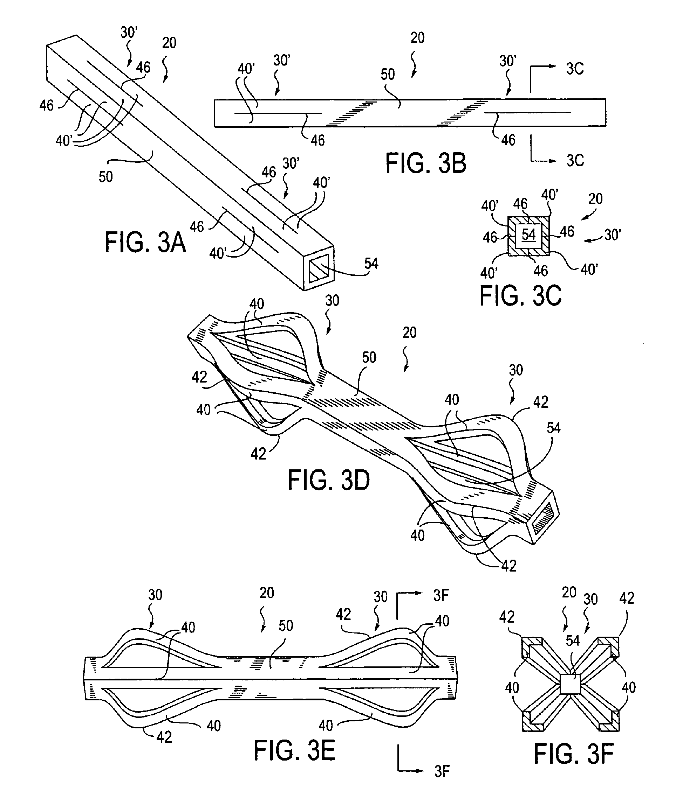

**FIGS. 3A -3F** show a fracture-stabilization device with a rectangular body and four radially expandable struts, each arising from a cut through a flat surface of the body and expanding with a leading sharp edge. **Fig 3A** is a perspective view of the body of the device. **FIG. 3B** is a side view of the body of the device showing slots forming the struts. **FIG. 3C** is a cross-sectional view of the device. **FIG. 3D** is a perspective view of the device after the struts have radially expanded. **FIG. 3E** is a side view of the device after the struts have radially expanded. **FIG. 3F** is an end view of the device after the struts have radially expanded.

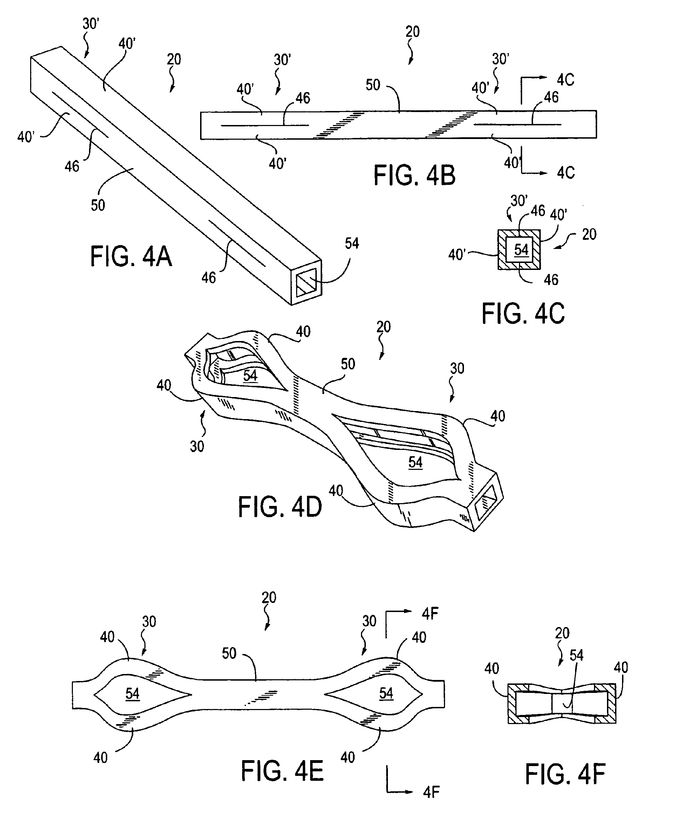

**FIGS. 4A -4F** shows a fracture-stabilization device with a rectangular body and two radially expandable struts arising from length-wise cuts in a flat surface of the body and expanding with a leading flat edge. **FIG. 4A** is a perspective view of the body of the device. **FIG. 4B** is a side view of the body of the device showing slots. **FIG. 4C** is a cross-sectional view of the device. **FIG. 4D** is a perspective view of the device after the struts have radially expanded. **FIG. 4E** is a side view of the device after the struts have radially expanded. **FIG. 4F** is an end view of the device after the struts have radially expanded.

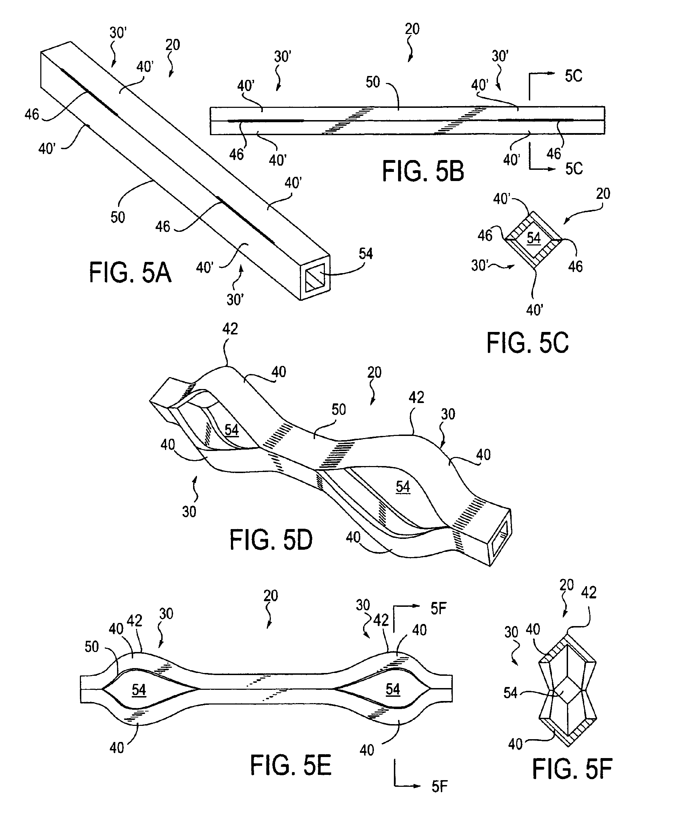

**FIGS. 5A -5F** show a fracture-stabilization device with a rectangular body and two radially expandable struts formed by length-wise cuts at a vertex of the rectangle, each strut expanding with a leading sharp edge. **FIG. 5A** is a perspective view of the body of the device. **FIG. 5B** is a side view of the body of the device showing slots to be cut from which struts will emerge. **FIG. 5C** is a cross-sectional view of the device. **FIG. 5D** is a perspective view of the device after the struts have radially expanded. **FIG. 5E** is a side view of the device after the struts have radially expanded. **FIG. 5F** is an end view of the device after the struts have radially expanded.

**FIG. 6** shows a single anchorable member with two radially opposed struts in an expanded configuration, the member being a component joinable with a connector portion and a second anchor to form a complete fracture fixation device.

**FIG. 7** shows a perspective view of a single anchorable member with three radially distributed struts in an expanded configuration, the member being a component that is joinable with a connector portion and a second anchor to form a complete fracture fixation device.

**FIG. 8** shows a perspective view of a single anchorable member with four radially opposed struts in an expanded configuration, the member being a component joinable with a connector portion and a second anchor to form a complete fracture fixation device, the anchorable member further including a central rod that maintains a continuous passageway with a connector in the fully assembled device. The connector portion and/or rod include holes from which a flowable bone cement may be ejected.

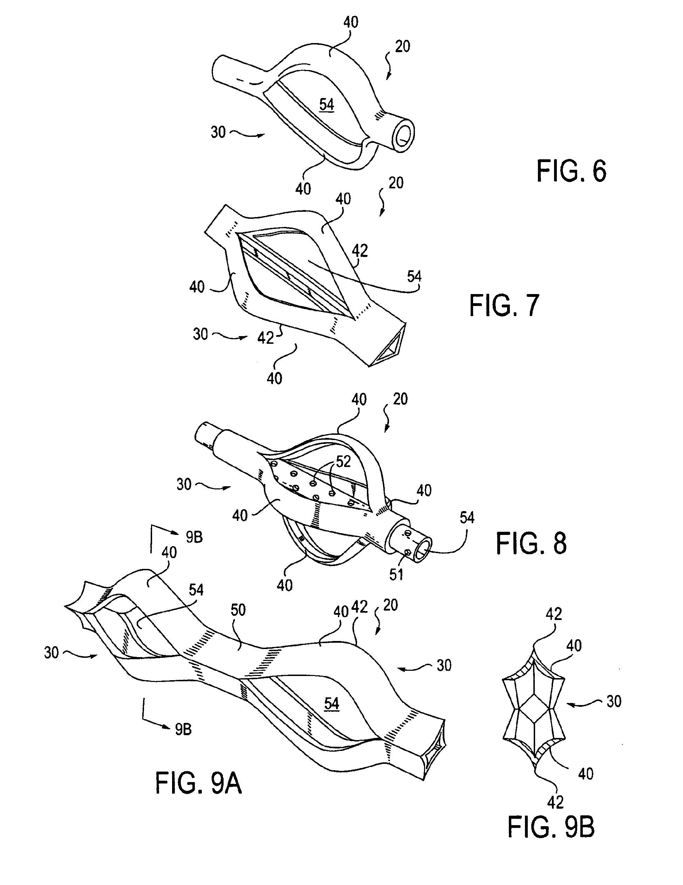

**FIGS. 9A and 9B** show a fracture-stabilization device with a rectangular body and two radially expandable struts emanating from length-wise cuts at a vertex of the rectangle. This device is similar to that depicted in **FIG. 5** except that the corners of the rectangle have been pinched or crimped in, giving the corner an angle more acute than 90 degrees. These acute corners become the leading edge of a strut as it expands, and in this embodiment the leading edge is particularly sharp. **FIG. 9A** is a perspective view of the body of the device. **FIG. 9B** is a partial view through an expanded struts.

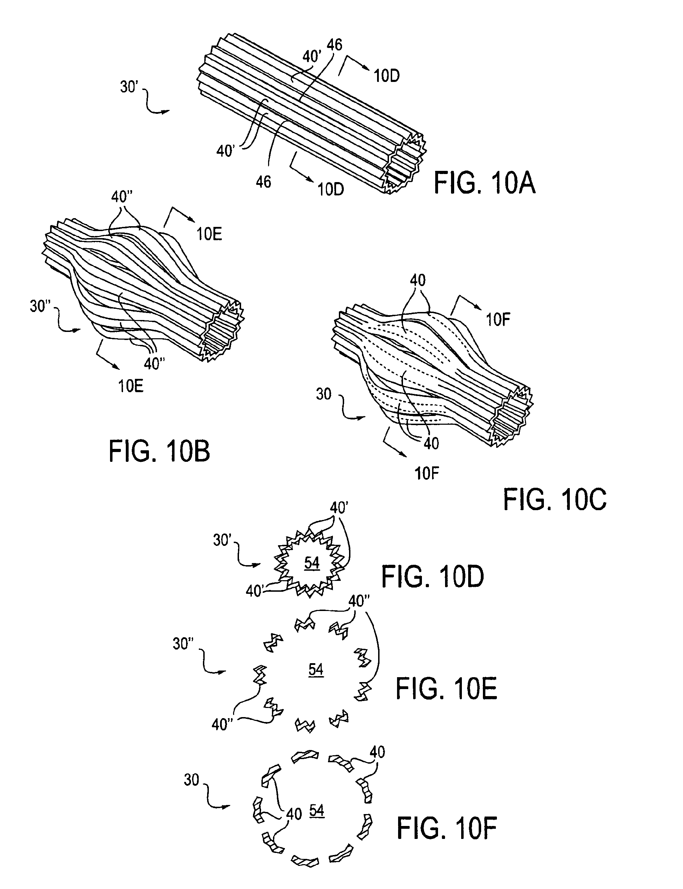

**FIGS. 10A - 10F** show one anchorable member of an embodiment of a fracture fixation device with a linearly corrugated surface, from which nine expandable struts emanate. **FIG. 10A** shows the body of the anchorable anchorable member in a linearly constrained, non-radially expanded configuration. Slots are present though not visible in the inner vertex of corrugations. **FIG. 10B** shows expansion of the expandable struts to a first position, which may either be a partial or fully self-expanded configuration. **FIG. 10C** shows expansion of the expandable struts to a second position, more expanded than the first position of **FIG. 10B. FIG. 10D** shows a linearly cross sectional view at position **10D** of **FIG. 10A****,** showing the corrugated nature of the body of the expandable member. **FIG. 10E** shows a linearly cross sectional view at position **10E** of **FIG. 10B****,** showing the M-shaped cross-sectional profile the expanded struts. **FIG. 10F** shows a linearly cross sectional view at position **10F** of **FIG. 10C****,** showing the flattened M-shaped cross-sectional profile the expanded struts.

**FIG. 11A** shows a fracture-stabilization device exploded into three parts, illustrating various dimensions of the device. **FIG. 11B** shows a cross section of the body of an anchorable member. **FIG. 11C** shows a cross section of the struts at their most expanded point. **FIG. 11D** shows a cross section of an alternative embodiment with three struts rather than four struts.

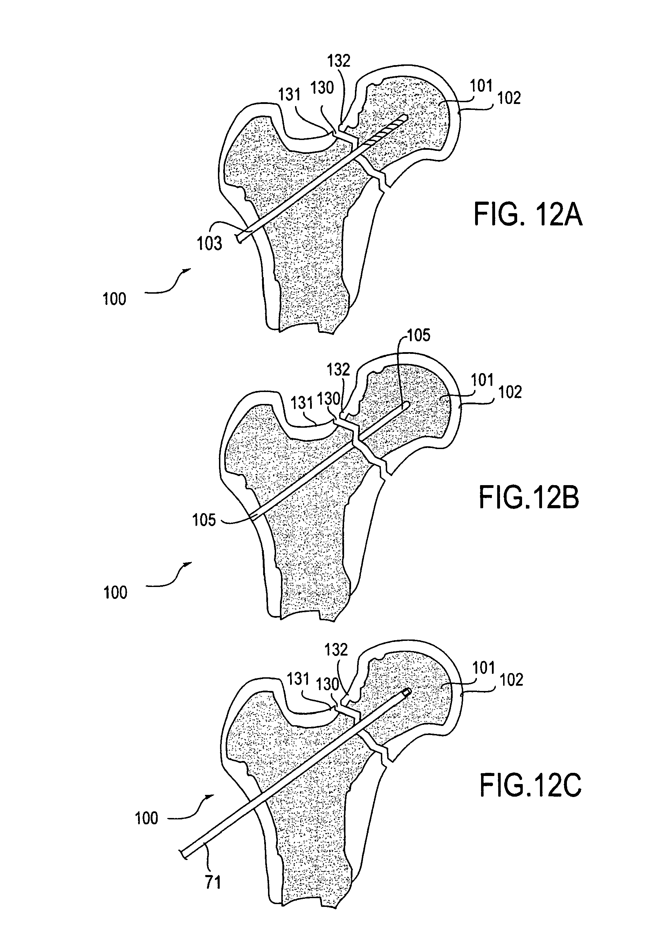

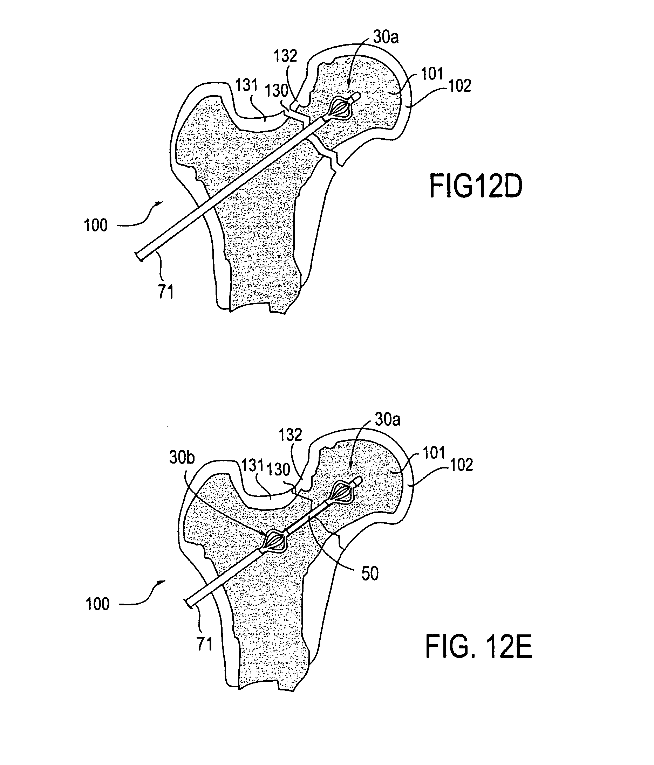

**FIGS. 12A -12E** illustrate deployment of a fracture-stabilization device into a hip bone, passing through a point just below the greater trochanter of the femur, across the fracture, and into the head of the femur. **FIG. 12A** shows a drill bit forming a passageway for the device. **FIG. 12B** shows the formed passageway prepared to receive the device. **FIG. 12C** shows a delivery device inserted into the passageway, positioning the distal anchorable member of a device. **FIG. 12D** shows the first or distal anchorable member after expansion of the struts. **FIG. 12E** shows the *device in situ,* the proximal or second expansion member after expansion of its struts, and after the two anchorable members of the device have been drawn together, tightening the fracture zone.

**FIG. 13** depicts two fracture-stabilization devices implanted into a fracture of a hip in location similar to that depicted in **FIG. 12****.**

**FIG. 14** depicts two fracture-stabilization devices implanted into a flat bone such as a skull plate, the devices substantially flat in their expanded profile, the expandable members having two struts.

**FIG. 15** shows one variations of a fracture-stabilization kit, the kit including an Allen head tool, a first and a second anchorable member, a connector, a delivery device, a container of flowable cement, a push rod for delivering a distal anchor, and a delivery rod for delivering a proximal anchor.

**FIGS. 16A -16O** illustrate a fracture fixation device in three conjoinable units (first and second anchorable members and a connector portion), as well as anchoring opposition rods, in various stages of assembly, but ultimately into a complete and implanted device. The device is shown variously both in isolation, *ex situ,* and as implanted, *in situ.* **FIG. 16A** shows the first anchorable member constrained in a linear configuration by a portion of the inserter, the anchorable member threadably-connected to the delivery device.

**FIG. 16B** shows the first anchorable member after the inserter (push or opposition rod portion) has been partially withdrawn, allowing the anchorable member to self-expand.

**FIG. 16C** shows the first anchorable member being further expanded by a mechanical assist, the opposition rod remaining engaged at the distal portion of the first expandable member, and being pulled proximally by the rod, which is still engaged at the distal end of the first anchorable member. This is an optional step; **FIG. 16D** continues as if this step had not been taken.

**FIG. 16D** shows the first anchorable member released from the linearly constraining opposition rod and in a self-expanded configuration, the rod now withdrawn from the first anchorable member.

**FIG. 16E** shows the first anchorable member *in situ* in the self-expanded configuration, as it's anchored in its expanded configuration.

**FIG. 16F** is an *in situ* view showing the connector portion being threadably connected to the first anchorable member, the connector being deployed by a delivery device, the delivery device threadably connecting the connector to the first anchorable member.

**FIG. 16G** shows first anchorable member and the connector now conjoined, and a second anchorable member now being brought into position to engage the connector portion by a delivery device with a rod constraining the anchorable member in its linear configuration.

**FIG. 16H** shows the second anchorable member now in contact with the connector, the member still being linearly constrained by the rod of the delivery device.

**FIG. 16I** shows the second anchorable member now released from its proximal attachment to the rod, and the anchorable member linearly contracted and radially expanded.

**FIG. 16J** is an *in situ* view showing the second anchorable member implanted and expanded, and the deploying rod now withdrawn.

**FIG. 16K** shows an Allen wrench connector deployer extending through the second anchorable member to engage the connector and beginning to rotate the connector with respect to the two anchorable members.

**FIG. 16L** shows the first and second anchorable members now drawn together by a turn-buckle rotation of the connector threadably engaged with both the first and second anchorable members.

**FIG. 16M** is an *in situ* view showing the fully assembled device in its anchoring configuration, the two anchorable members drawn together to the desired degree toward the connector, and the deployment device having been withdrawn.

**FIG. 16N** shows an injector tube inserted into the device and a flowable cementing composition being injected through the passageway extending there through, the cement composition being emitted into the space within the expanded struts of the anchoring members, and through holes in the connector to emerge into available space peripheral to the connector.

**FIG. 16O** shows the device, the cement inserter removed, the device now fully implanted, and stabilized by the cement now hardened.

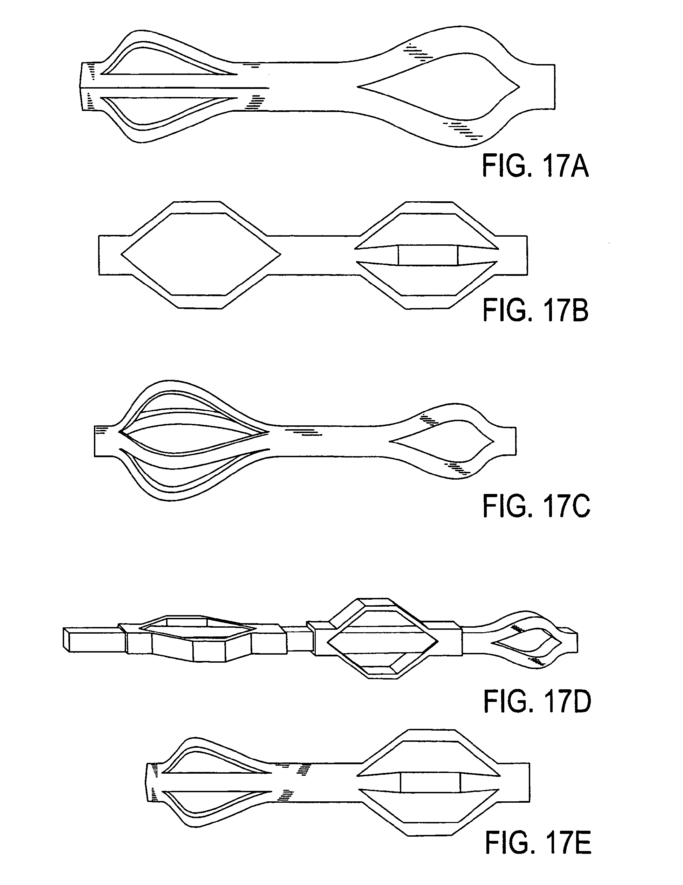

**FIGS. 17A -17E** show various embodiments of fracture fixation devices that have dissimilar first and second anchoring or anchorable members for custom fitting into fracture sites. **FIG. 17A** is a device with a three-strut anchorable member and a two-strut anchorable member, in each case that struts curvilinear and asymmetrically bowed. **FIG. 17B** is a device with a two-strut anchorable member and a four-strut anchorable member, in case the struts are symmetrically bowed and having substantially straight segments. **FIG. 17C** is a device with a four-strut anchorable member that is significantly larger than its two-strut companion. **FIG. 17D** shows a device with three anchorable members, each member having two struts, the members expanding in different radial orientations, two with substantially straight segments in the struts, and a third with curvilinear struts. FIG. 17E is a device with an anchorable member having two asymmetrically bowed struts and a central hollow rod and a second anchorable member with four symmetrically bowed struts and without a central rod.

### DETAILED DESCRIPTION OF THE INVENTION

Described herein are bone fracture fixation systems and devices, and methods of using them to repair fractures. The figures illustrate various embodiments of the system. Although the description specifies the use of embodiments of the fracture fixation system to repair a fracture of the femoral head, the devices, systems and methods described herein may be used to repair other fractures as well. Embodiments of the devices provided herein may be applied to a wide variety of bones and to various fractures that they may incur. Sizes and specifics of device conformation and configuration are readily varied, and devices may be assembled so as to fit the specifics of a particular fracture site. Further, the devices may be applied to regions of bone that include cancellous bone, cortical bone, or both types of bone.

In general, the fracture-fixation devices described herein include two anchorable (or anchoring) members connectable or connected by a connector. These anchorable members include self-expanding structures such as struts. As will be seen, struts may be highly variable in form, and may include for example, outwardly expanding structures that lead with flat, rounded, or sharp cutting edges. In some fracture sites, a cutting edge may be preferred as a way to cut into the bone most effectively to form an anchor, and in other sites, it may be preferred to lead with a flat of rounded surface that can provide more substantial outward support to a bone when the device is in its final anchoring position. Various embodiments and features of the system will be described with general references to **FIGS. 1**-**17,** and FIGS. 1-17 will be detailed individually in greater detail thereafter.

A system for stabilizing or fixing a bone fracture **20** may include two anchorable members 30 with an intervening connector piece **50.** Anchorable members **30** can also be referred to as a first member **30a** and second member **30b.** Typically, the first member **30a** is distal with respect to the second or thus proximal member **30b,** distal referring to a position furthest from the delivery device (*e.g*., deepest within a fracture site from the perspective of a physician implanting the device) or from the perspective of a delivery (or deployment) device that positions the device within the site of fracture. The anchorable members typically have two configurations; one configuration is substantially collapsed, which may be linear in orientation. This is the non-anchoring (or delivery) configuration of the member in which it may be deployed and positioned in a fracture site. The second configuration is an anchoring (or expanded) configuration, which typically includes a radially expanded structure. An anchorable member in a constrained or non-expanded configuration may be labeled as member 30' (30 prime).

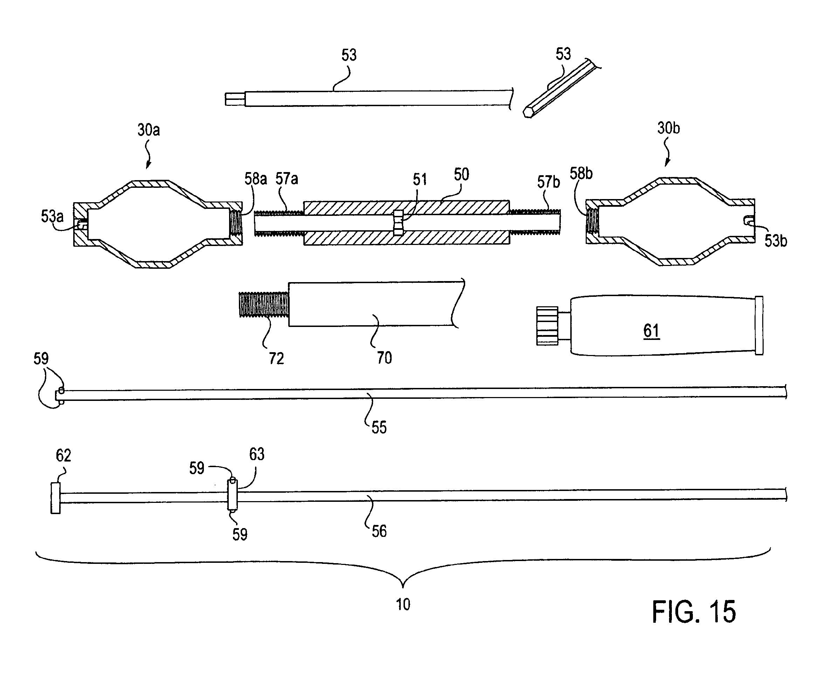

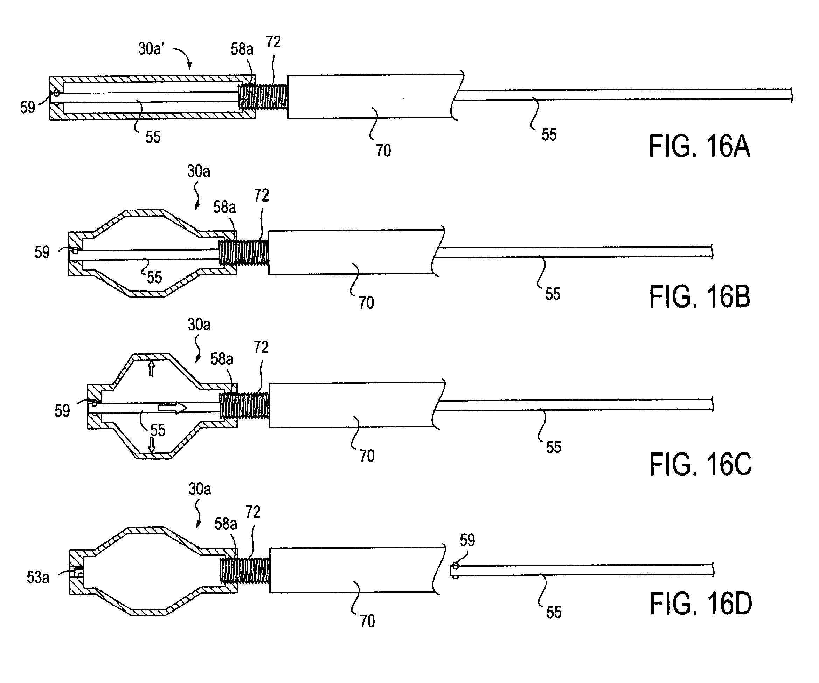

The first or distal anchorable member 30a, the connector 50, and the second or proximal anchorable member 30b are all separate pieces that are conjoinable. In some embodiments of the fracture fixation device, the invention includes a kit of parts that may be assembled into a complete device 20 before implantation in a fracture site, or such parts may not be fully assembled until the time when they are being positioned within the fracture site. See **FIG. 15** for an embodiment of a kit of parts. **FIGS. 16A -16O** illustrate one variation of a method of inserting a first anchorable member, a connector, and a second anchorable member in order to assemble a complete device. In some variations, a connector is a connector region extending from one or both anchorable members.

The connector and both anchorable members are separate. They may be connected in any appropriate manner. For example, they may be threaded (*e.g*., connected by screwing), or may be slidably connected (*e.g*., one or more anchorable members may slide over the connector region) that can interlock.

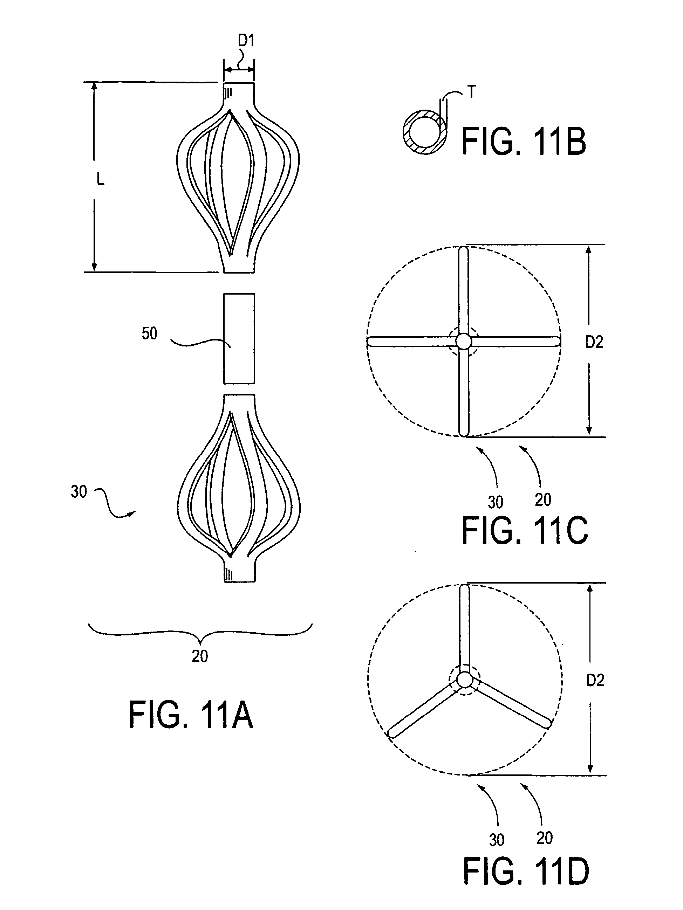

The dimensions of anchorable members **30** of a fracture stabilizing device **20** may be selected according to their intended site of use. The exemplary dimensions provided here are to help in providing an understanding, and are not intended to be limiting. **FIGS. 11A-11D** show an embodiment of the device **20** and provides visual reference for various dimensions, and is described in further detail below. As noted above, the fracture fixation device 20 may be embodied as a kit of parts. These parts may have a modular character in that, in spite variations in size and form of some regions, there may be limited variation in some dimensions. For example, the diameter of the body may have a limited number of sizes so that parts are readily conjoinable around common features, particularly points of threadable connections, as between a connector and anchorable members, and as in the size of the lumen extending through a connector and as such lumen or rod may further extend through anchorable members. A device 20 assembled from various parts could have identical first and second anchorable members, or the members could be dissimilar. The great variety of devices that may be generated from such a system allows for custom fitting of a device to the dimensions of a fracture and the surround fracture regions; a few such exemplary devices with dissimilar first and second anchorable members are depicted in **FIGS. 17A - 17E****.**

Anchorable members 30 (and possibly connector 50) may be formed from any appropriate material. In particular, shape-memory materials. Anchorable members may be formed by "prebiasing" them into a shape such as an expanded (anchoring) shape. In some variations, components of the fracture-fixation device are formed at least partially from a resiliently deformable material such as a plastic, metal, or metal alloy, stainless steel, for example, or a shape memory (and super-elastic) metal alloy such as Nitinol. A detailed description of materials that may be suitable for the fabrication of the present fracture fixation device may be found in US Patent Application No. 11/468,759 (US 2007/067034).
In typical embodiments of an anchorable member, the preferred state of the member is that of the radially-expanded anchoring configuration. In these embodiments, the unexpanded configuration that is appropriate for deployment and initial positioning within a fracture site is a constrained configuration.

Embodiments of the invention may constrain an anchorable member 30' in at least two ways, which will be described in greater detail below. Briefly, one approach is that of confining the member within an enclosing cannula or sleeve 71 that physically prevents radial expansion. A delivery device including a cannula or sleeve is shown in **FIGS. 12A-12E****,** wherein a fracture fixation device configured as a single conjoined unit prior to delivery is implanted in a fracture zone of a femoral head. In these embodiments, the delivery device may include a push rod, to distally eject a fracture fixation device. In some variations, the device, or regions of the device (*e.g*., the anchorable members) are place under tension by the delivery device to prevent them from expanding. Radial expansion may shorten or contract the anchorable members of device. Thus, a delivery device may include one or more attachment sites to constrain the anchorable members from expanding. For example, a delivery device may apply tension to the anchorable members through a rod (*e.g*., a length-constrainment rod) extending distally from a delivery or deployment device **70.** The rod may prevent shortening of length and radial expansion of anchorable members. The rod may be slidable within the delivery device, but can be held (*e.g*., locked) in an extended position to prevent deployment of the anchorable member. An example of a delivery device including a rod for applying or maintain tension is depicted in **FIGS. 15** **and** **16A** **-160,** and described in considerable detail further below.

An anchorable device **20** may include two anchorable members **30** and a connector **50,** and each of these components includes a passageway or channel **54** there through, that forms a continuous passageway **54** though the fracture-fixation device. The passageway **54** may form a lumen through which a rod **57** may be inserted, and through which a flowable cementing or bone-filling material **61** may be conveyed. The passageway may also be a hollow tube **54** that may form a strengthening structural element for the device **20** as a whole. In some embodiments, only the connector portion includes hollow tube **54;** in other embodiments, the hollow tube is included as a structural feature of one or more of the anchorable members. The connector and/or tube **54** also may also be configured so that the anchorable members **30** may be moved closer or further apart from each other. For example, the connector and/or tube may be threaded and rotation of either the connector or one or more of the anchorable members may draw the members closer together.

In its constrained (delivery) configuration, an anchorable member **30'** may be in the form of a substantially hollow tube. In some variations, the cross-section of the fracture fixation device is substantially circular or oval (as in **FIGS. 1** **and** **2**), particularly the expandable members. In some variations, it is a sided-structure, *e.g*., having three sides, four sides, or more than four sides (as in **FIGS. 4****,** **5****, and** **9**). In an embodiment with four sides, a rectangular configuration may have four sides of equal length. Further variations of the cross sectional profile occur in other embodiments. For example, the vertices or corners of a sided-embodiment may be pinched or crimped in (**FIG. 9A and** 9B), this configuration may create a more acute cutting edge on the struts as they undergo their self-expansion upon release of the device from constraint. In other embodiments, the surface may be substantially round in profile, but embellished with linear corrugation, as **show in** **FIGS. 10A -10C****. In this configuration, the linear folds of the struts may impart** strength to the struts that remains even in the expanded configuration of the struts.

As described in US Patent Application no. 11/468,759 (Pub No. US 2007/0067034 A1) and US Provisional Patent Application No. 60/916,731 (WO 2008/137192), slots or slits 46 may be cut lengthwise in a tube to form nascent struts **40**. With metallurgical methods well known in the art such as heat treatment, the struts **46** may be configured into a preferred configuration such as a bow. In some device embodiments, the configuration of bowed struts may be linearly symmetrical or substantially symmetrical (as shown in **FIGS. 5****,** **10****, and** **15**), and in other embodiments, the bow may be asymmetrical (as shown in **FIGS. 1-4****),** with the maximal expanded portion skewed either toward the distal or proximal end of an anchorable member. Other configurations of symmetrical and asymmetrical struts may also be used.

An anchorable member **30** having three struts comprising the body **45 of** device 20 typically has a triangular cross section, the struts formed by slots cut through the surface of each of the three sides. In an embodiment where the triangle of the cross-section is equilateral, the struts are radially distributed equally from each other, with 120 degrees separating them (see alternative embodiment in **FIG. 11****).** In other embodiments, where the triangle of the cross sections is not an equilateral triangle, the radial angles of struts may include two that are equal, and a third angle that is not equal to the other two There may be some benefits associated with anchorable member embodiments with three struts compared with four or more struts. The struts formed are wider, and thereby may be stronger than members having four struts emanating from a device body of the same diameter.

In some four-strut variations, the body 45 of the device 20 is either square or circular in cross section, and the four struts 40 emanating from the body are typically equally spaced apart at 90 degrees, or they may be radially distributed such that the angles formed include two angles greater than 90 degrees and two angles less than 90 degrees. A body 45 with a square cross section typically is appropriate to support struts that are spaced apart by 90 degrees, the strut-forming slots positioned centrally lengthwise along the body **(****FIGS. 4A - 4F****).** This configuration also imparts a 90 degree leading edge on struts **40** formed therefrom, such an edge being useful in cutting through bone. In many embodiments of the invention, efficiency in cutting through bone, either or both cortical bone or cancellous bone, is advantageous. Cutting may separate bone mass to allow strut movement through bone with minimal compression of bone, and thus minimal disturbance of bone tissue in regions adjacent to the path of separation. Bone (particularly cortical bone) may be cut only slightly, and may serve to help anchor the device in or to the bone. In other embodiments, it may be desirable that struts **40** have a surface that presents a flat face for bone support, *e.g*., expandable members having a circular cross section (as illustrated in **FIGS. 1A -1F** **and** **2A - 2F**).

In some variations, the anchorable member includes only two struts. In these variations, the **45** of a device **30** may be circular **(****FIG.** 6) or square **(****FIGS. 4A - 4F****)** in cross section. In embodiments having a square cross section, lengthwise slots 46 may be made at opposite vertices of the square, in which case the two struts formed therefrom have a 90 degree leading edge **(****FIGS. 5A - 5F****).** For example, a body having a circular cross section may include lengthwise slots **46** that may be made at radially opposite positions, in which case the two struts formed therefrom have a broad leading edge **(****FIG. 6****).** In some embodiments of a fracture-fixation device **20,** a broad leading edge may be beneficial if the leading edge is intended to provide support to a bone surface from within.

As mentioned, the struts **40** may be formed by cuts or slots **46** in the body of the device **20** and may include a sharp cutting edge **42** useful for cutting, scoring or securing to bone (either cancellous bone **101** or cortical bone **102)** as the struts radially expands upon being released from constraint **(****FIGS. 3A - 3F**, **5A - 5F**, and **9A - 9B****).** A sharp edge may be derived from a vertex or corner of the device body as seen in cross section. Thus, for example, a rectangular body or a triangular body can generate struts with a sharp leading edge as the struts expand. In typical embodiments, for example, where struts are formed from a the body of an anchorable device with a rectangular cross section, cuts in the metal to create slots are made in the central portion of sides of the rectangle, and struts **40** are formed at the vertices of the rectangle. Thus, in some embodiments, the cutting edge **42** of a strut **40** may have a leading angle of about 90 degrees. In other embodiments of an anchorable member **30** with a rectangular cross sectional profile, the vertices of the rectangle may be crimped or pinched in order to create corner angles that are more acute than 90 degrees **(****FIG. 9A and 9B****).** In embodiments such as these, the cutting edge **42** or a strut **40** may have a leading angle more acute than 90 degrees. In embodiments of an anchorable member **30** with an (equilateral) triangular cross section, the vertices of the triangle have an angle of 60 degrees, and thus struts **40** formed from such vertices have a cutting edge **42** with an angle of 60 degrees.

In some embodiments of a fracture fixation device **20,** the first anchorable member **30a** and the second anchorable member **30b** are identical (*e.g*., **FIGS. 1 - 6****).** In other embodiments of a fracture fixation device **20,** the first **30a** and second **30b** anchorable members are dissimilar **(****FIGS. 17A -17E****).** Fracture-fixation device may include anchorable members that are different in size (*e.g*., length of body **45,** length of struts **40,** differences in diameter of the body **45),** different in the radial expansiveness of the released configuration of struts **40,** different with regard to the symmetry or asymmetry of bowed struts **40,** or different in any other anchorable member parameter. By such variations in form of the two anchorable members **30,** a fracture fixation device **20** may be tailored to suit the particular dimensions of a target fracture site. As described above, a device **20** may be further tailored or fitted to a target fracture site by any of the variations in size provided by embodiments of anchorable members **30** and their components, such as struts **40** or connector **50.**

Some embodiments of a fracture fixation device **20** may include an internal anchorable member within an external anchorable member **(****FIGS. 2A - 2F****).** The benefit provided by this general configuration is that it provides more surface area (*e.g*., twice as much) for anchoring within a given anchoring volume of bone than does a single anchoring member. Typically, the number of struts in the companion internal and external bodies are the same, and are radially staggered with respect to each other, so that the struts of the inner body may emerge in the spaces between the struts of the outer body. The struts of the inner and outer bodies may be of about the same length and bowed outwardly to about the same degree, as they are in **FIGS. 2A - 2F****.** In other embodiments, the struts of the inner body may be shorter in length, or bowed outward to a lesser degree than the struts of the outer body.

A fracture stabilizing system **10** may included one or more delivery devices. By way of example, a delivery device may be a sleeve or cannula **71** which constrains embodiments of device **20** for deployment **(****FIGS. 12A -12E****).** Deployment occurs by means of a push rod extending distally in the delivery device to a point of contact on the proximal surface of the second or proximal anchorable member **30b.** By pushing the device **20** distally and at the same time withdrawing the cannula from an implantation site, the first or distal anchorable member **30a** emerges from the cannula and self-expands as it is released from the lateral or circumferential constraints of the cannula. As the cannula is withdrawn further in the proximal direction from an implantation site and simultaneously continuing to push the device distally out of the cannula, a connector portion **50** and a second or proximal anchorable member **30a** emerge in sequence. As the second anchorable member is released from the circumferential constraints of the cannula, it self-expands, as did the first anchorable member.

A second exemplary delivery device **70** illustrated herein generally constrains the fracture-fixation device to a linear configuration and prevents expansion of struts by applying tension across at least a portion of the device to prevent contraction of shortening of the body of the device (as in **FIGS. 16A -16O****).** Embodiments of this delivery device may be similar to embodiments of delivery devices disclosed in detail in US Provisional Patent Application No. 60/916731, filed on May 8, 2007 (WO 2008/137192).

A fracture-fixation device may be delivered by providing a delivery device that constrains the anchorable members from contraction. The delivery device can be used to sequentially expand a first anchorable member, and a second anchorable member, either sequentially or simultaneously- The device may be inserted with all of the components of the fracture-fixation device attached (*e.g*., fully assembled) or with them in components that are joined after (or during) delivery.

As described above, some embodiments of device **20** may be fabricated from a superelastic shape memory alloy such as Nitinol, in which case struts **40** may be configured to self-expanding when released from constraint in a radially non-expanded (or linear form). When implanted in bone, particularly in hard cortical bone, expansion of struts may be resisted by surrounding bone. Facing such resistance, expandable struts **40** may not expand to their full potential. Inasmuch as greater anchoring stability is associated with full radial expansion, it may be advantageous to mechanically assist struts in their expansion. Additional mechanical expansion may be achieved by drawing the distal and proximal ends of anchorable members closer together. **FIG. 16C** shows an exemplary mechanism by which mechanical force is applied to partially expanded struts **40** in order to assist in their full expansion.

Following implantation of a fracture fixation device, a flowable bone filling composition or cement 61 such as PMMA (polymethylmethacrylate) may injected into the fracture region through a trocar and cannula system into the passageway **54** of a device **20.** There are many suitable materials known in the art for filling in vacant spaces in bone, some of these materials or compositions are biological in origin and some are synthetic, as described in US Patent Application No. 11/468,159 (WO 2007/067034)**.**
From the passageway, the material flows into the open space within the anchorable members and to some degree, into the peripheral area surrounding the device. The flowable cementing material may contain radiopaque material so that when injected under live fluoroscopy, cement localization and leakage can be observed.

Another example of bone cementing material is provided by a ceramic composition including calcium sulfate calcium hydroxyapatite, such as Cerament™ as manufactured by BoneSupport AB (Lund, Sweden). Ceramic compositions provide a dynamic space for bone ingrowth in that over time, they resorb or partially resorb, and as a consequence provide space for ingrowth of new bone. Bioactive agents may also be included in a cementing composition, such as octeogenic or osteoinductive peptides, as well as hormones such at parathyroid hormone (PTH). Bone Morphogenetic Proteins (BMPs) are a prominent example of effective osteoinductive agents, and accordingly, a protein such as recombinant human BMP-2 (rbBMP-2) may included in an injected bone-filling composition. In this particular context, BMPs promote growth of new bone into the regions in the interior of the expanded struts and around the periphery of device 20 in general, to stabilize the device within new bone. A more fundamental benefit provided by the new bone growth, aside from the anchoring of the device **20,** is simply the development of new bone which itself promotes healing of a fracture. Antibiotics may be included, particularly when there is reason to believe that the fracture site may have been infected. With the inclusion of bioactive agents such as bone growth or differentiation factors, or antibiotic or other anti-infective agents, embodiments of the fracture fixation device become more than a fracture stabilizing or fixation device, as such embodiments take on the role of an active therapeutic or drug delivery device. In general, any appropriate flowable material may be injected into the passageway formed through the fracture-fixation device. In some variations the device (*e.g*., the proximal end of the fracture-fixation device) may be adapted to receive a device for delivering flowable material.

Examples of fracture-fixation devices, system and methods of using them are provided below, including methods of implanting the device across a fracture to stabilize it and to promote its healing, as particularly detailed in **FIGS. 1-17****.**

For example, **FIGS. 1A - 1F** provide views of a fracture-stabilization device 20 with a circular body having a lumen 54 and two anchorable members **30a, 30b,** each with four radially expandable struts 40', the struts having a flat expanding surface, and a connector portion **50.** **Fig 1A** is a perspective view of the body of the device. **FIG. 1B** is a side view of the body of the device showing slots 46 to be cut from which struts will emerge. FIG. 1C is a cross-sectional view of the device. **FIG. 1D** is a perspective view of the device after the struts **40** have radially expanded. **FIG. 1E** is a side view of the device after the struts have radially expanded. **FIG. 1F** is an end view of the device after the struts have radially expanded. A number of structural features of embodiments of the dual-anchoring system **20** described herein, such as slots **46,** struts **40,** and anchorable members in general, as well as methods of delivery and implantation are similar to features of a vertebral body stabilization device with a single anchorable ember, as described in US Patent Application No. 11/468,759 (WO 2007/067034), which may help in the understending of the present invention.

**FIGS. 2A -2F** provide views of an internal-external, or double-bodied, fracture-stabilization device, the outer body **20** surrounding an internal body 21. Each body has a lumen 54 and two anchorable members **30,** each with four expandable struts 40¹, the struts 41 of the internal body and the struts **40** of the external body staggered with respect to each other, and a connector portion 50. FIG. 2A is a perspective view of the body of the device. **FIG. 2B** is a side view of the body of the device showing slots 46 to be cut from which struts will emerge. **FIG.** 2C is a cross-sectional view of the device. **FIG. 2D** is a perspective view of the device after the struts **40** have radially expanded. **FIG. 2E** is a side view of the device after the struts have radially expanded. **FIG. 2F** is a cross-sectional view through the struts of the device after the struts have radially expanded.

**FIGS.** 3A **3F** provide views of a fracture-stabilization device **20** with a rectangular body having a lumen **54** and two anchorable members **30,** each with four radially expandable struts **40¹,** each emanating from a slot 46 cut through a flat surface of the body and expanding with a leading sharp edge **42,** and a connector portion **50.** **Fig 3A** is a perspective view of the body of the device. **FIG. 3B** is a side view of the body of the device showing slots **46** to be cut from which struts will emerge. **FIG. 3C** is a cross-sectional view of the device. **FIG. 3D** is a perspective view of the device after the struts **40** have radially expanded. **FIG. 3E** is a side view of the device after the struts have radially expanded. **FIG. 3F** is a cross-sectional view through the struts of the device after the struts have radially expanded.

**FIGS. 4A -4F** provide views of a fracture-stabilization device **20** with a rectangular body having a lumen **54** and two anchorable members **30,** each with two radially expandable struts **40¹** emanating from length-wise cuts in a flat surface of the body and expanding with a leading flat edge, and a connector portion **50.** **FIG. 4A** is a perspective view of the body of the device. **FIG. 4B** is a side view of the body of the device showing slots 46 to be cut from which struts will emerge. **FIG. 4C** is a cross-sectional view of the device. **FIG. 4D** is a perspective view of the device after the struts **40** have radially expanded. FIG. 4E is a side view of the device after the struts have radially expanded. **FIG. 4F** is a cross-sectional view through the struts of the device after the struts have radially expanded.

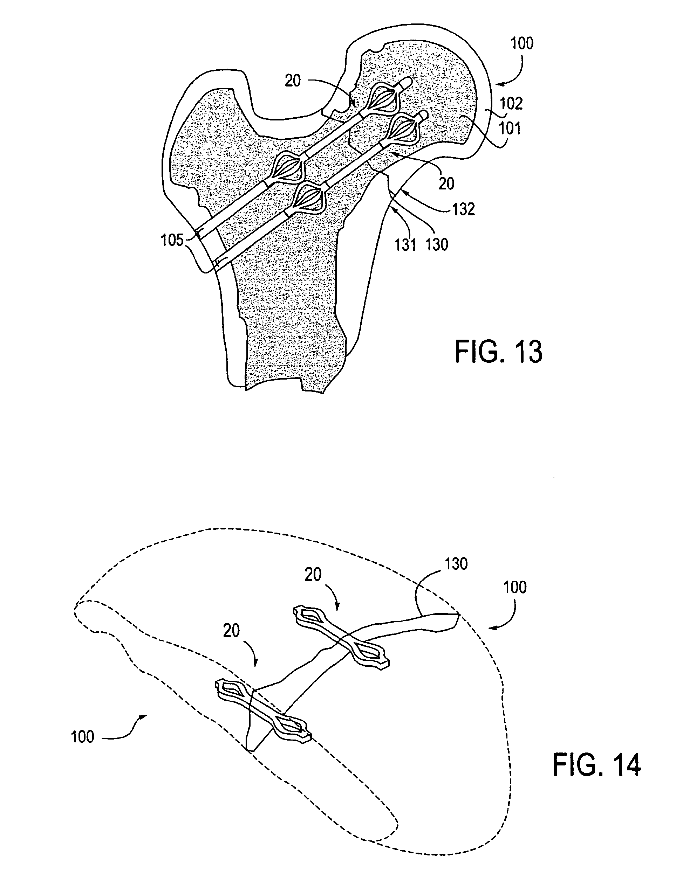

**FIGS. 5A -5F** provide views of a fracture-stabilization device **20** with a rectangular body having a lumen **54** and two anchorable members **30**, each with two radially expandable struts **40¹** emanating from length-wise cuts at a vertex of the rectangle, each strut expanding with a leading sharp edge **42,** and a connector portion 50. **FIG. 5A** is a perspective view of the body of the device. **FIG. SB** is a side view of the body of the device showing slots **46** to be cut from which struts will emerge. **FIG. 5C** is a cross-sectional view of the device. **FIG. 5D** is a perspective view of the device after the struts have radially expanded. **FIG. 5E** is a side view of the device after the struts 40 have radially expanded. **FIG. 5F** is cross-sectional view of through the struts of the device after the struts have radially expanded. Device embodiments such as these depicted in **FIG. 5****,** **FIG. 4****,** and **FIG. 9** with two radially expandable struts may be particularly advantageous for fixing fractures in a flat bone such as a skull plate **(****FIG.14****)** or in any bone or fracture site that is small, or has a narrow planar constraint.

As mentioned above, although the examples shown in FIGS. 1A and 2A are fracture fixation devices that are integrally formed, the anchorable regions of the invention are separate and attachable via the connector region. Further, one or more attachment regions may be included for attachment to a delivery device (including both distal and proximal attachment sites), and attachment to a length-adjusting device (for changing the spacing between the anchorable members), or attachment to a source of flowable material (*e.g*., cement). Attachment sites may be threaded attachment sites, interlocking attachment sites (*e.g*., keyed attachment sites), gripping attachment sites, or any appropriate releasable attachment site.

**FIGS. 6 - 8** show exemplary anchorable members **30** which may be understood as components of a complete double-anchored device 20, these single anchorable members being presented to exemplify particular features comparative way. **FIG. 6** provides a view of a single anchorable member **30** with two radially opposed struts **40** in an expanded configuration, the member being a component joinable with a connector portion and a second anchor to form a complete fracture fixation device. **FIG. 7** provides a view of a single anchorable member **30** with three radially distributed struts **40** in an expanded configuration, the member being a component joinable with a connector portion and a second anchor to form a complete fracture fixation device.

**FIG. 8** provides a view of a single anchorable member **30** with four radially opposed struts **40** in an expanded configuration, the member being a component joinable with a connector portion and a second anchor to form a complete fracture fixation device, the anchorable member further including a central rod or tube **54** that forms a continuous passageway with a connector in the fully assembled device. In some variations, the connector is the central tube **54** shown, and the anchorable members 30 may be slidable thereon. The anchorable members may be locked into position. In some variations, the connector does not lock to the anchorable members. The connector portion and/or the rod may include holes **52** from which a flowable bone cement may be ejected. Lumen **54** as seen in **FIG. 8** in the form of a central rod extending through the anchorable member **30** may also be understood as to include the contiguous open space, in general, within the interior of expanded struts **40** as depicted in **FIG. 6** and **FIG. 7****.**

**FIGS. 9A and 9B** provide views of a fracture-stabilization device **20** with a rectangular body and two anchorable members **30**, each with two radially expandable struts emanating from length-wise cuts at a vertex of the rectangle. This device is similar to that depicted in **FIG. 5** except that the corners of the rectangle have been pinched or crimped in, giving the corner an internal angle more acute than 90 degrees. These acute corners become the leading and cutting edge **42** of a strut **40** as it expands, and in this embodiment the leading edge is particularly sharp. **FIG. 9A** is a perspective view of the body of the device. **FIG. 9B** is a view of one strut of the device after radial expansion.

**FIGS. 10A - 10F** show a portion of one anchorable member of an embodiment of a double-anchored fracture fixation device with a linearly corrugated or crenellated surface, from which nine expandable struts **40'** emanate. **FIG. 10A** shows the anchorable anchorable member **30'** in a linearly constrained, non-radially expanded configuration. Slots **46** are present in the inner vertex of corrugations. **FIG. 10B** shows the anchorable member **30"** with expansion of the struts **40"** to a first position, which may either be a partial or fully self-expanded configuration, depending on the preferred configuration of the heat-treated shape memory metal. **FIG. 10C** shows expansion the anchorable member **30** and the expandable struts **40** to a second position, more expanded than the first position of **FIG. 10B. FIG. 10D** shows a radial cross sectional view of anchorable member **30'** at position **10D** of **FIG. 10A****,** showing the corrugated nature of the body of the anchorable member. **FIG. 10E** shows a radial cross sectional view of anchorable member **30"** at position **10E of** **FIG. 10B**, showing the M-shaped cross-sectional profile the expanded or partially-expanded struts **40".** **FIG. 10F** shows a radial cross sectional view of anchorable member **30** at position **10F** of **FIG. 10C**, showing the flattened M-shaped cross-sectional profile of fully expanded struts **40**.

**FIGS. 11A - 11D** show one example of a fracture-stabilization device that has been exploded into three parts, as well as cross sectional views of the body of the device, and of the anchorable members in their expanded configuration. This figure may illustrate the location of various dimensions of the device. Dimensions of anchorable members **30** of a fracture stabilizing device **20** may be chosen according to their intended site of use. The exemplary dimensions provided here are to help in providing an understanding of the invention, and are not intended to be limiting. For example, in some embodiments, the length L of the body **45** of an anchorable member when the struts **30** are in the radially expanded configuration may vary from about 7.5 mm to about **48** mm, and in particular embodiments, from about 24 mm to about 40 mm. In other embodiments, for particular applications, the length of the body may be less than 7.5 mm or greater than 48 mm. The thickness **T** (**FIG. 11B**) of the tube wall of a tubular body **45** may vary from about 0.2 mm to about 2.5 mm, and in typical embodiments is about 0.5 mm in thickness. The outside diameter **D1** of the body of the device in its linear configuration may vary. In one variation, the outer diameter varies between about 1 mm to about 8 mm in diameter. **FIG. 11D** shows a cross sectional view of an alternative embodiment with three struts, radially distributed at 120 degrees, is included to convey the applicability of this diameter measurement even when struts do not form a straight-line diametric structure as can four struts. In the context of a released or anchoring configuration of an anchorable device 30, the struts 40 may expand to a maximal radial distance (**FIGS 11C** and **11D**) from about 3.5 mm to about 22 mm, to create a maximal diameter **D2** (extrapolating the strut profiles to form a circle enclosing the maximal points of expansion) of about 7.5 mm to about 44 mm. In other embodiments, for particular application to particular fracture sites, the maximal expansion diameter may be less than 4 mm or greater than 25 mm.

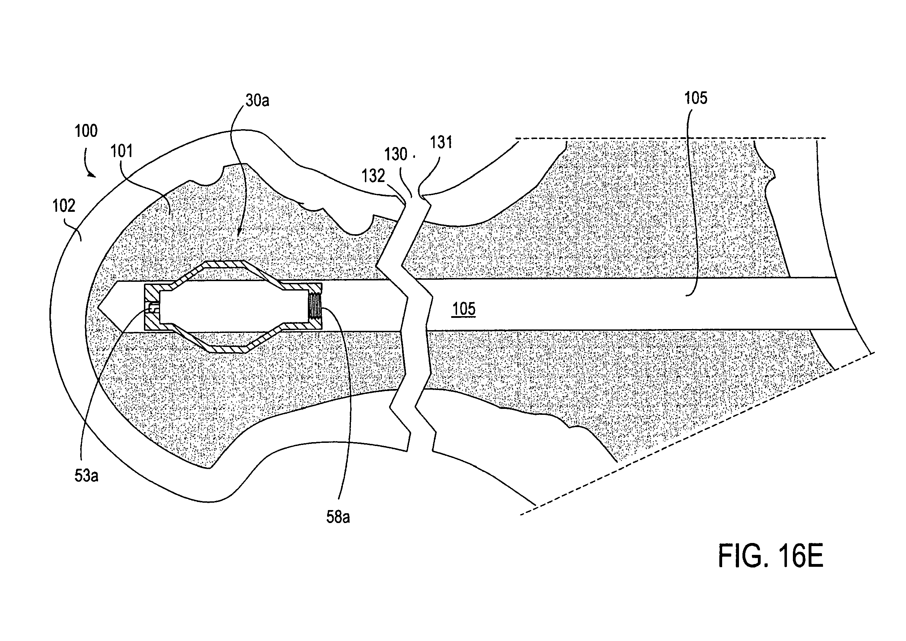

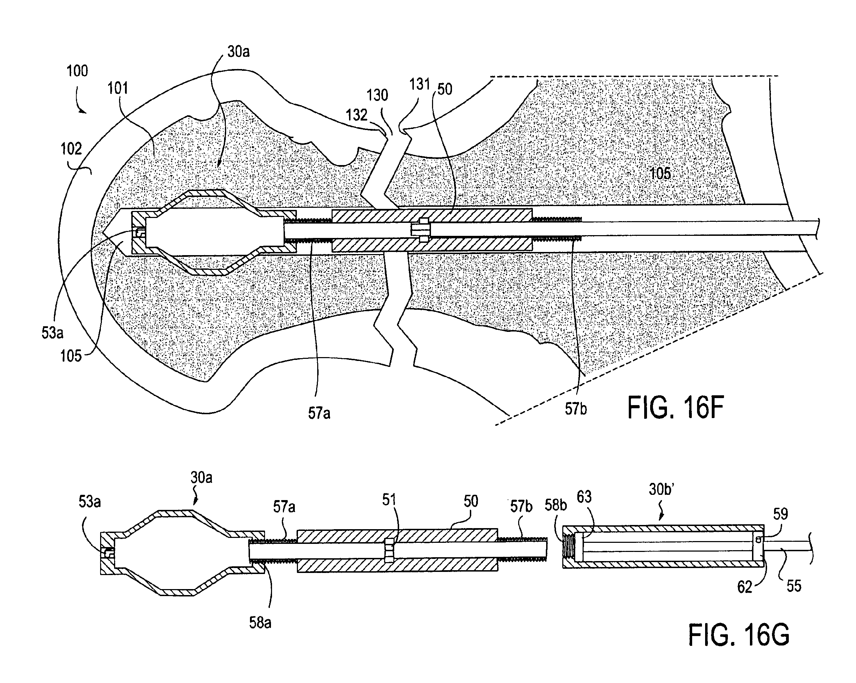

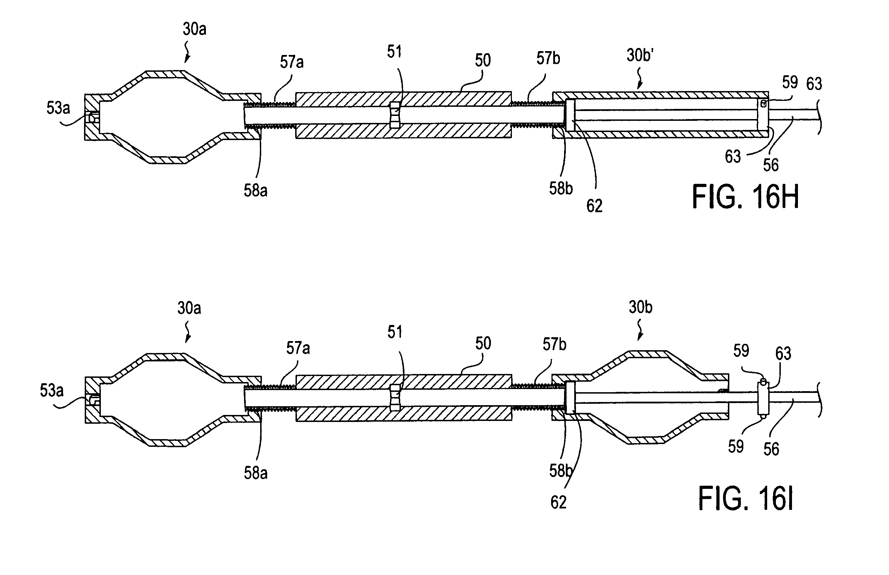

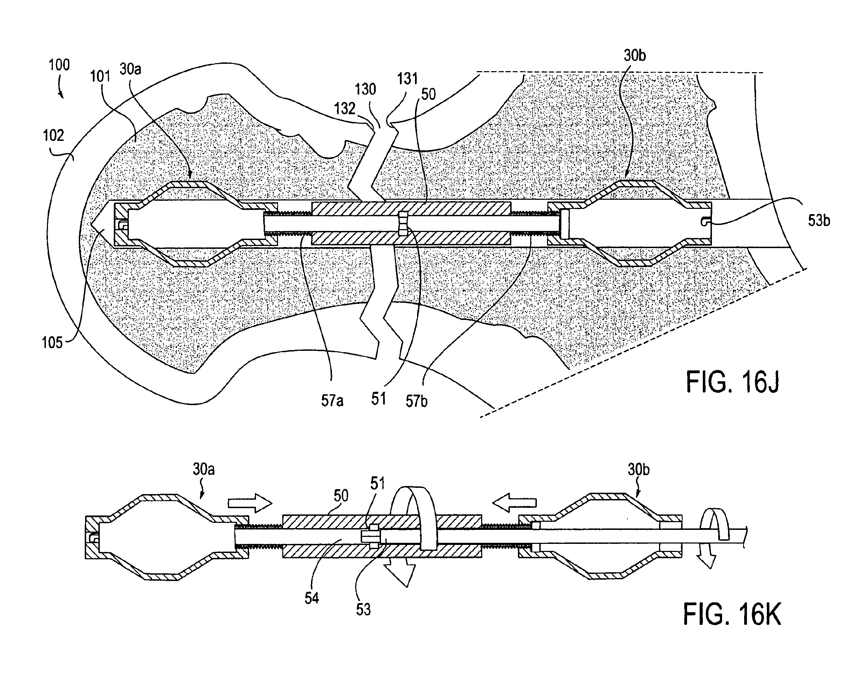

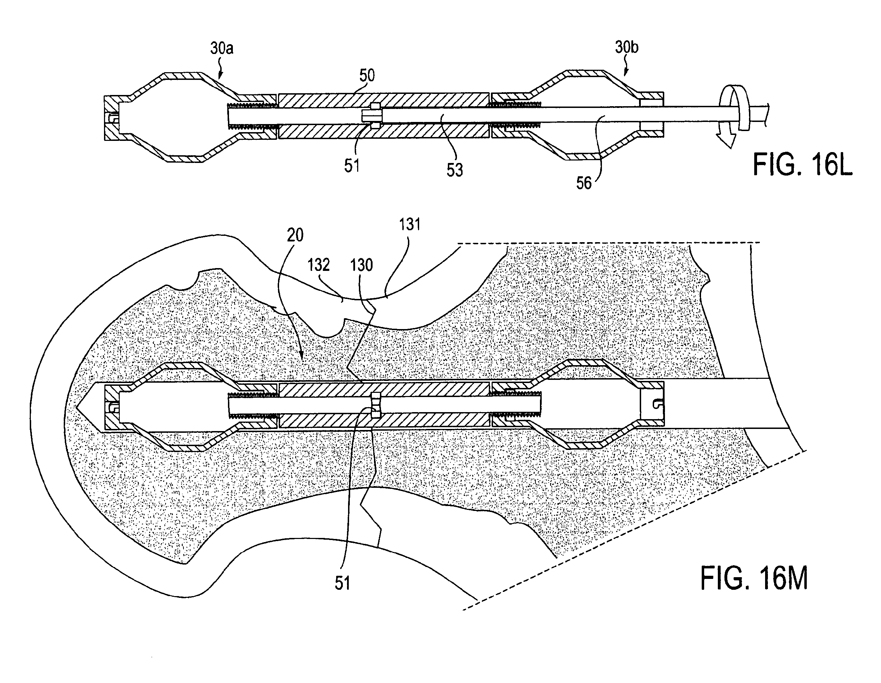

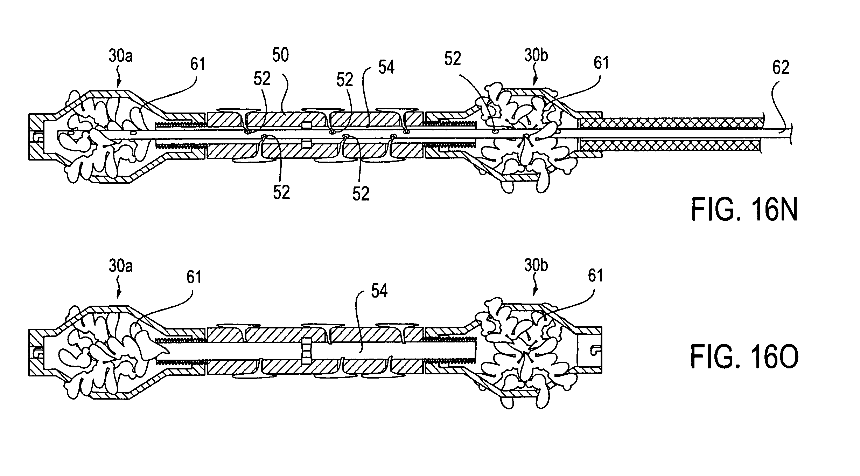

**FIGS. 12A - 12E** show views of the deployment of a fracture-stabilization device into a hip, passing through a point just below the greater trochanter of the femur, through a proximal region of bone **131**, across the fracture **130**, and into a distal region of bone **132** within the head of the femur. **FIG. 12** also shows the distribution of cancellous bone **101** and cortical or dense bone **102** within the femur. **FIG.12A** shows a drill bit 103 forming a passageway for the device; such drilling typically occurs after aligning the fracture so as to restore the fractured bone to its natural position, or to a position that best approximate the natural position. Such alignment may be attained by methods well known in the art, including the use of a goniometer. **FIG. 12B** shows the formed passageway **105** prepared to receive the device. **FIG. 12C** shows a delivery device **71**, in this example, a cannula or a device with a distal portion that includes a sleeve that radially envelopes the device, being inserted into the passageway, and positioning the distal anchorable member of a device. **FIG.12D** shows the first or distal anchorable member after expansion of the struts after the first or distal anchorable member **30a** of the device has been partially pushed out of the distal end of the cannula **71**, while at the same time, the cannula has been partially withdrawn from the implant site. The exemplary device in this series of figures is being inserted in its complete form, i.e., with the first anchorable member 30a, the connector 50, and the second anchorable member 30b already either conjoined prior to implantation, or the device as a whole fabricated as single integrated device. In some variations of the fracture-fixation devices that are delivered by a cannula, the anchorable members of the device maybe self-expand upon release from the radial constraints of the cannula. Further, in such embodiments, the first anchorable member expands first, and the second expandable member expands second. **FIG. 12E** shows the device in situ, the proximal or second expansion member after its struts have expanded, and after the two anchorable members of the device have been drawn together, tightening the fracture zone **130**. Details of drawing two anchorable members together after implantation of a device are shown in **FIG. 16**, as described below.

Some fractures may benefit from the implanting of more than one fracture-stabilization device. In these instances, each device needs to have preparatory drilling to form a passageway and implanting in a manner similar to that detailed for a single device as in FIG. **12**. **FIG. 13** depicts two fracture-stabilization devices implanted into a fracture of a hip in location similar to that depicted in **FIG. 12**.

**FIG. 14** depicts two fracture-stabilization devices implanted into a flat bone 100 such as a skull plate; the devices **20** are substantially flat in their expanded profile as the expandable members each have two coplanar struts (such as those depicted in **FIGS. 4****,** **5****, or** **9**).

**FIG. 15** depicts an embodiment of a fracture-stabilization system that is in the form of a kit **10**, the kit including an Allen head tool **53** shown in a side view and a perspective view, a first anchorable member **30a** and a second anchorable member **30b**, a connector **50**, a delivery device **70**, a container of a flowable bone filling composition **61**, and an applicator, including a first rod **55** for engaging the first or distal anchor **30a**, and a second (outer) rod **56** for engaging the second or proximal anchor **30b**. The two rods of the delivery system may constrain the anchorable members from expanding during deployment. After delivery, one or both rods may be withdrawn, allowing anchorable members to contract and radially self-expand into anchoring configurations.

The delivery device **70** in this example has a distal threaded portion **72** that engages threads **58a** on the first anchorable member **30a**. The first anchorable member **30a** has a connecting region (rod engaging feature **53a**) that engages plug **59** on rod **55**. The second anchorable member has a connecting region (rod engaging **53b**) that engages plug **59** on rod **56**. Rod **56** further has a stop bar **62** that meets the interior of the distal end of the second anchorable member and a plug mount **63** with plugs **59** that engage the proximal end of the second anchorable member. Rods **55** and **56** may both be considered embodiments of a length-constraining rod, which may constrain the length (in this case, prevent contraction) of an anchorable member, by engaging in a releasable way either or both the proximal or distal portion of an anchorable member in such a way that contraction of the member is prevented. The releasable-engagement means that interact between an anchorable member and a length-constraining rod may be of any suitable type. In the particular embodiments shown, the feature on the rods are male plugs that can rotate into female slots within the anchorable members, but the male-female orientation may be reversed in some embodiments, or more generally be of any suitable mechanism. Connector **50** has threaded portion **57a** that engages threads **58a** on first anchorable member **30a**, and connector **50** also has threads **57b** that engage threads **58b** on second anchorable member **30b**. Connector **50** further has an Allen head female feature **51** that engages the male head on Allen tool **53**. The threads **57a** and **57b** of the connector and their respectively engaging threads on the respective anchorable members are configured oppositely such that the connector **50** acts like a turnbuckle when turned by the Allen tool **53,** and can thus pull the anchorable members together or extend them further apart.

**FIGS. 16A -16O** illustrate of a fracture fixation device in three conjoinable units (first and second anchorable members and a connector portion), and describe assembly and insertion of one variation of the fracture-fixation device. In this sequence of figures, the device is shown variously both in isolation, *ex situ,* and as implanted across a fractured region within the neck of a femur. **FIG. 16A** shows the first anchorable member **30a'** in a constrained configuration, held by the delivery device including opposition rod **55**. The anchorable member is shown threadably-connected to the outer rod of the delivery device **70** (also a hollow element), and the connector element is also secured to the delivery device, so that the distal anchorable member is held in the collapsed configuration as tension is applied.

**FIG. 16B** shows the first anchorable member after opposition rod **55** has been partially withdrawn, releasing the applied tension and allowing the anchorable member to at least partially self-expand.

**FIG. 16C** shows the first anchorable member being further expanded by a mechanical assist. The opposition rod **55** has been re-engaged (or has remained engaged) at the distal portion 59 of the first expandable member **30a**, and the distal portion is being pulled proximally by rod **55**. This is an optional step in the implantation of the device, and an analogous step may be taken with regard to the second or proximal anchorable member. Although the anchorable members are self-expanding, and expand to a preferred configuration when their expansion is unimpeded, when implanted in bone, such expansion can meet variable amounts of resistance. For this reason, under some conditions, it may be desirable to mechanically assist in expansion of the struts of the anchoring configuration of an anchorable member. An analogous mechanical expansion step and a tool for such has been described in US Patent Application No. 11/468,759.

**FIG. 16D** continues as if the step shown in FIG. **16C** had not been taken, and shows the first anchorable member released from the linearly constraining opposition rod **55** and in a self-expanded configuration, rod **55** now withdrawn from the first anchorable member **30a**. The second (proximal) anchorable member may then be applied by detaching the threaded delivery device, and inserting a connector, as shown in FIG. 15 **50**. The connector can be threaded or otherwise attached to the end of the first anchorable member. A second anchorable member can them be inserted by holding it in tension and attaching it (*e.g*., via threads) to the proximal end of the connector. Once it has been connected, the tension may be released, and it may be allowed to self-expand. The connector can be adjusted to change the spacing between the expanded anchorable members, as described above. During this process the fracture-fixation device has maintained a central passageway.

**FIG. 16E** shows a similar first anchorable member **30a** *in situ* in the self-expanded configuration, as it is anchored in its expanded configuration. It can be seen that the anchorable member positioned in a region of bone **132** that is proximal to fracture **130**, with respect to the path of entry **105**. The anchorable member is embedded in cancellous bone region **101** of a femoral head **100**, which is encased in cortical bone **102**.

**FIG. 16F** is an *in situ* view showing the connector portion **50** being threadably connected to the first anchorable member **30a**, the connector being deployed by a delivery device, the delivery device threadably connecting the connector **50** to the first anchorable member **30a**.

**FIG. 16G** shows first anchorable member **30a** and the connector **50** now conjoined, and a second anchorable member **30b'** now being brought into position to engage the connector portion **50** by a delivery device with a rod **55** constraining the anchorable member **30b'** in its linear configuration.

**FIG. 16H** shows the second anchorable member **30b'** now in contact with the connector **50**, the member still being linearly constrained by the rod **56** of the delivery device. More specifically, it can be seen that stop bar **62** and the plugs **59** on mount **63** of rod **56** are preventing the linear contraction (and consequent radial expansion) of anchorable member **30b'**.

**FIG. 16I** shows the second anchorable member **30b** now released from its proximal attachment to the rod (the rod 56 has been rotated, releasing plugs **59** from their engagement site at the proximal end of anchorable member **30b**), and the anchorable member **30b** has now linearly contracted and radially expanded.

The exemplary deployment sequence just described is one in which the first anchorable member expands first, after implantation as a single piece, and then a connector is added, and then the second anchorable member, which then radially expands. Other embodiments of the inventive method include implantation of a device that is assembled *in situ,* but delaying the expansion of the first anchorable member until assembly of the device is complete, and then expanding the two anchorable members simultaneously, or nearly simultaneously. In other arrangements as described above and shown in **FIGS. 12A -12E****)**, a fully assembled or integrally formed device is implanted and the anchorable member then each radially-expanded synchronously.

**FIG. 16J** is an *in situ* view showing the second anchorable member **30b** now implanted and expanded, and the deploying rod 56 now withdrawn from the implant site. Both anchorable members are now expanded in their anchoring configuration, however the fracture gap **131** has not yet been tightened by the drawing closer of the two anchorable members **30a** and **30b**.

**FIG. 16K** shows an Allen wrench connector deployer **53** extending through the second anchorable member **30b** to engage the connector at Allen female feature **51** within connector **50** and beginning to rotate the connector with respect to the two anchorable members, drawing them closer together, as indicated by the directional yarrows.

**FIG. 16L** shows the first **30a** and second **30b** anchorable members now drawn together by a turn-buckle rotation of the connector **50** threadably engaging both the first and second anchorable members in a turnbuckle manner. The pulling together or approximating of the anchorable members may be complemented by the reverse action, a distraction or separating of the anchorable members, as may be required or desired in some procedures. Further, such manipulations may be done before expansion of one or both of the anchorable members.

**FIG. 16M** is an in *situ* view showing the fully assembled device 20 in its anchoring configuration, the two anchorable members drawn together to the desired degree toward the connector, the fracture regions **131** and **132** also drawn together, and the deployment device having been withdrawn.

**FIG. 16N** shows an injector tube **62** connected to the proximal end device 20, engaging a connection on anchorable member **30b**, and injecting a flowable cementing composition **61** through the passageway **54** extending through the device. The cementing composition **61** is being emitted into the space within the expanded struts of the anchoring members **30a** and **30b**, and through holes **52** in connector **50** to emerge into available space within bone that is peripheral to the connector.

**FIG. 16O** shows the device, the cement inserter removed, the device now fully implanted, and stabilized by the cement **61** now hardened.

**FIGS. 17A -17E** show various embodiments of fracture fixation devices that have dissimilar first and second anchors for custom fitting into fracture sites. **FIG. 17A** shows a device with a three-strut anchorable member and a two-strut anchorable member, in each case that struts curvilinear and asymmetrically bowed. **FIG. 17B** shows a device with a two-strut anchorable member and a four-strut anchorable member, in case the struts are symmetrically bowed and having substantially straight segments. **FIG. 17C** shows a device with a four-strut anchorable member that is significantly larger than its two-strut companion. **FIG. 17D** shows a device with three anchorable members, each member having two struts, the members expanding in different radial orientations, two with substantially straight segments in the struts, and a third with curvilinear struts. **FIG**. **17E** shows a device with an anchorable member having two asymmetrically bowed struts and a central hollow rod and a second anchorable member with four symmetrically bowed struts and without a central rod.

Although the fracture fixation devices described herein typically include two anchorable (expandable) regions separated by a connector region, other variations are encompassed by this disclosure, including devices having more than two anchorable regions. For example, a series of interconnected expandable regions could form a fracture-fixation device. In addition, the connector regions could be formed of bendable, or rotatable material. In some variation the connector region or component is adjustable to shorten or lengthen the spacing between them without rotating them. For example, the connector region may be an interlocking telescoping region.

While the methods and devices have been described in some detail here by way of illustration and example, such illustration and example is for purposes of clarity of understanding only. It will be readily apparent to those of ordinary skill in the art in light of the teachings herein that certain changes and modifications may be made thereto. The invention is defined by the following claims.

## Claims

1. A system for stabilizing a bone fracture, comprising:
a first self-expanding anchorable member (30a) and a second selt-expanding anchorable member (30b), each member having a central passageway, each member having a constrained non-anchoring configuration and a released anchoring configuration, the first self-expanding anchorable member (30a) having a distal connecting region (53a) and a proximal internally threaded region (58a), and the second self-expanding anchorable member having a distal internally threaded region (58b) and a proximal connector region (53b); and
a connector (50) having a central passageway, the connector (50) having a distal threaded region (57a) configured to attach to the proximal region (58a) of the first self-expanding anchorable member (30a) and a proximal threaded region (57b) configured to attach the distal region (58b) of the second self-expanding anchorable member (30b), such that the central passageways of the anchorable members (30a,b) and the connector (50) form a continuous passageway; and
wherein the first self-expanding anchorable member (30a) and the second self-expanding anchorable member (30b) and the connector (50) are separable and independently implantable into a bone.

2. The system of claim 1, wherein the released anchoring configuration includes a radially expanded structure.

3. The system of claim 1 or 2, wherein the released anchoring configuration of the self-expanding anchorable members (30a,b) includes at least one cutting surface.

4. The system of any one of the preceding claims, wherein the released anchoring configuration of the self-expanding anchorable members (30a,b) includes radially expanded struts.

5. The system of claim 4, wherein the radially-expanded struts form a symmetrical bow or an asymmetrical bow.

6. The system of claim 5, wherein the radially-expanded struts form an asymmetrical bow having its greatest radial diameter distributed proximally.

7. The system of any one of the preceding claims, wherein the passageways of the first self-expanding anchorable member (30a), the connector (50), and the second self-expanding anchorable member (30b) are adapted to convey a flowable material (61).

8. The system of any one of the preceding claims, wherein the connector (50) includes holes (52) adapted to allow egress of a flowable material (61).

9. The system of any one of the preceding claims, wherein the distal and proximal threaded regions (57a,b) of the connector (50) are threaded oppositely such that the connector (50) acts like a turnbuckle such that rotation of the connector changes the distance between the two self-expanding anchorable members.

10. The system of any one of the preceding claims, further comprising a delivery device (70) and a rod (55) for positioning the first anchorable member into a bone fracture site, said delivery device (70) being configured to be releasably attach to the proximal internally threaded region (58a) on the first anchorable member, the rod (55) being further configured to attach to the distal connecting region (53a) of the first self-expanding anchorable member (30a).

11. The system of claim 10, wherein the delivery device (70) comprises a sleeve (71) that radially encloses the first self-expanding anchorable member (30a), the connector (50), and the second self expanding anchorable member (30b).

12. The system of claim 11, further comprising a push rod (56) configured to extend from the proximal connector region (53b) of the second self-expanding anchorable member (30b) and releasably attach to the proximal connector region (53b) of the second self-expanding anchorable member (30b).

13. The system of any one of the preceding claims, wherein the connector (50) further comprises an Allen head female feature (51) for engaging the male head on an Allen tool, thereby rotating the connector (50) and changing the distance between the first and second self-expanding anchorable members (30a,b).

14. The system of any one of the preceding claims, wherein the first and second self-expanding anchorable members (30a,b) are different shapes and/or sizes.

15. The system of claim 14, wherein the first and second self-expanding anchorable members (30a,b) are different in size, different in radial expansiveness of the released configuration, and/or different with regard the symmetry or asymmetry.

## Patentansprüche

1. System zum Stabilisieren eines Knochenbruchs, das Folgendes umfasst:
ein erstes selbstausdehnendes verankerbares Element (30a) und ein zweites selbstausdehnendes verankerbares Element (30b), wobei jedes Element einen mittigen Durchgang hat, wobei jedes Element eine eingeengte nicht verankernde Konfiguration und eine freigegebene verankernde Konfiguration hat, wobei das erste selbstausdehnende verankerbare Element (30a) eine distale Verbinderregion (53a) und eine proximale mit Innengewinde versehene Region (58a) hat und das zweite selbstausdehnende verankerbare Element (30b) eine distale mit Innengewinde versehene Region (58b) und eine proximale Verbinderregion (53b) hat,
einen Verbinder (50), der einen mittigen Durchgang hat, wobei der Verbinder (50) eine distale mit Gewinde versehene Region (57a), die dafür konfiguriert ist, an der proximalen Region (58a) des ersten selbstausdehnenden verankerbaren Elements (30a) befestigt zu werden, und eine proximale mit Gewinde versehene Region (57b), die dafür konfiguriert ist, die distale Region (58b) des zweiten selbstausdehnenden verankerbaren Elements (30b) zu befestigen, hat derart, dass die mittigen Durchgänge der verankerbaren Elemente (30a, b) und des Verbinders (50) einen durchgehenden Durchgang bilden, und
wobei das erste selbstausdehnende verankerbare Element (30a) und das zweite selbstausdehnende verankerbare Element (30b) und der Verbinder (50) getrennt und unabhängig in einen Knochen implantiert werden können.

2. System nach Anspruch 1, wobei die freigegebene verankernde Konfiguration eine radial ausgedehnte Struktur einschließt.

3. System nach Anspruch 1 oder 2, wobei die freigegebene verankernde Konfiguration der selbstausdehnenden verankerbaren Elemente (30a, b) wenigstens eine Schneidfläche einschließt.

4. System nach einem der vorhergehenden Ansprüche, wobei die freigegebene verankernde Konfiguration der selbstausdehnenden verankerbaren Elemente (30a, b) radial ausgedehnte Streben einschließt.

5. System nach Anspruch 4, wobei die radial ausgedehnten Streben einen symmetrischen Bogen oder einen asymmetrischen Bogen bilden.

6. System nach Anspruch 5, wobei die radial ausgedehnten Streben einen asymmetrischen Bogen bilden, der seinen größten radialen Durchmesser proximal verteilt hat.

7. System nach einem der vorhergehenden Ansprüche, wobei die Durchgänge des ersten selbstausdehnenden verankerbaren Elements (30a), des Verbinders (50) und des zweiten selbstausdehnenden verankerbaren Elements (30b) dafür eingerichtet sind, ein fließfähiges Material (61) zu befördern.

8. System nach einem der vorhergehenden Ansprüche, wobei der Verbinder (50) Löcher (52) einschließt, die dafür eingerichtet sind, ein Austreten eines fließfähigen Materials (61) zu ermöglichen.

9. System nach einem der vorhergehenden Ansprüche, wobei die distale und die proximale mit Gewinde versehene Region (57a, b) des Verbinders (50) mit entgegengesetztem Gewinde versehen sind derart, dass der Verbinder (50) wie ein Spannschloss wirkt derart, dass eine Drehung des Verbinders den Abstand zwischen den zwei selbstausdehnenden verankerbaren Elementen verändert.

10. System nach einem der vorhergehenden Ansprüche, das ferner eine Zuführungseinrichtung (70) und einen Stab (55) zum Anordnen des ersten verankerbaren Elements in einer Knochenbruchstelle umfasst, wobei die Zuführungseinrichtung (70) dafür konfiguriert ist, lösbar an der proximalen mit Gewinde versehenen Region (58a) an dem ersten verankerbaren Element befestigt zu werden, wobei der Stab (55) ferner dafür konfiguriert ist, an der distalen Verbindungsregion (53a) des ersten verankerbaren Elements (30a) befestigt zu werden.

11. System nach Anspruch 10, wobei die Zuführungseinrichtung (70) eine Hülse (71) umfasst, die das erste selbstausdehnende verankerbare Element (30a), den Verbinder (50) und das zweite selbstausdehnende verankerbare Element (30b) radial umschließt.

12. System nach Anspruch 11, das ferner einen Schiebestab (56) umfasst, der dafür konfiguriert ist, sich von der proximalen Verbinderregion (53b) des zweiten selbstausdehnenden verankerbaren Elements (30b) aus zu erstrecken und an der proximalen Verbinderregion (53b) des zweiten selbstausdehnenden verankerbaren Elements (30b) befestigt zu werden.

13. System nach einem der vorhergehenden Ansprüche, wobei der Verbinder (50) ferner ein Innensechskantkopf-Merkmal (51) umfasst, um den Steckkopf an einem Sechskantwerkzeug in Eingriff zu nehmen, wodurch der Verbinder (50) gedreht und der Abstand zwischen dem ersten und dem zweiten selbstausdehnenden verankerbaren Element (30a, b) verändert wird.

14. System nach einem der vorhergehenden Ansprüche, wobei das erste und das zweite selbstausdehnende verankerbare Element (30a, b) unterschiedliche Formen und/oder Größen haben.

15. System nach Anspruch 14, wobei das erste und das zweite selbstausdehnende verankerbare Element (30a, b) unterschiedlich in der Größe, unterschiedlich in der radialen Ausdehnbarkeit der freigegebenen Konfiguration und/oder unterschiedlich in Bezug auf die Symmetrie oder Asymmetrie sind.

## Revendications

1. Système pour stabiliser une fracture osseuse, comprenant :
un premier élément ancrable auto-expansible (30a) et un second élément ancrable auto-expansible (30b), chaque élément comportant une voie de passage centrale, chaque élément comportant une configuration de non-ancrage restreinte et une configuration d'ancrage libérée, le premier élément ancrable auto-expansible (30a) comportant une région de raccord distale (53a) et une région proximale filetée intérieurement (58a), et le second élément ancrable auto-expansible comportant une région distale filetée intérieurement (58b) et une région de raccord proximale (53b) ; et
un raccord (50) comportant une voie de passage centrale, le raccord (50) comportant une région filetée distale (57a) configurée pour se fixer à la région proximale (58a) du premier élément ancrable auto-expansible (30a) et une région filetée proximale (57b) configurée pour se fixer à la région distale (58b) du second élément ancrable auto-expansible (30b), de sorte que les voies de passage centrales des éléments ancrables (30a, b) et du raccord (50) forment une voie de passage continue ; et
dans lequel le premier élément ancrable auto-expansible (30a) et le second élément ancrable auto-expansible (30b) et le raccord (50) sont séparables et indépendamment implantables dans un os.

2. Système selon la revendication 1, dans lequel la configuration d'ancrage libérée comprend une structure expansée radialement.

3. Système selon la revendication 1 ou 2, dans lequel la configuration d'ancrage libérée des éléments ancrables auto-expansibles (30a, b) comprend au moins une surface de coupe.

4. Système selon une quelconque des revendications précédentes, dans lequel la configuration d'ancrage libérée des éléments ancrables auto-expansibles (30a, b) comprend des entretoises expansées radialement.

5. Système selon la revendication 4, dans lequel les entretoises expansées radialement forment une courbure symétrique ou une courbure asymétrique.

6. Système selon la revendication 5, dans lequel les entretoises expansées radialement forment une courbure asymétrique dont le diamètre radial le plus important est distribué de façon proximale.

7. Système selon une quelconque des revendications précédentes, dans lequel les voies de passage du premier élément ancrable auto-expansible (30a), du raccord (50), et du second élément ancrable auto-expansible (30b) sont adaptées pour transporter un matériau liquide (61).

8. Système selon une quelconque des revendications précédentes, dans lequel le raccord (50) comprend des orifices (52) adaptés pour permettre la sortie d'un matériau liquide (61).

9. Système selon une quelconque des revendications précédentes, dans lequel les régions filetées distale et proximale (57a, b) du raccord (50) sont filetées de façon opposée de sorte que le raccord (50) agisse comme un tourniquet pour que la rotation du raccord change la distance entre les deux éléments ancrables auto-expansibles.

10. Système selon une quelconque des revendications précédentes, comprenant en outre un dispositif d'apport (70) et une tige (55) pour positionner le premier élément ancrable dans un site de fracture osseuse, ledit dispositif d'apport (70) étant configuré pour être fixé de façon libérable à la région proximale filetée intérieurement (58a) sur le premier élément ancrable, la tige (55) étant en outre configurée pour se fixer à la région de raccord distale (53a) du premier élément ancrable auto-expansible (30a).

11. Système selon la revendication 10, dans lequel le dispositif d'apport (70) comprend un manchon (71) qui enferme radialement le premier élément ancrable auto-expansible (30a), le raccord (50), et le second élément ancrable auto-expansible (30b).

12. Système selon la revendication 11, comprenant en outre une tige de poussée (56) configurée pour s'étendre à partir de la région de raccord proximale (53b) du second élément ancrable auto-expansible (30b) et se fixer de façon libérable à la région de raccord proximale (53b) du second élément ancrable auto-expansible (30b).

13. Système selon une quelconque des revendications précédentes, dans lequel le raccord (50) comprend en outre un accessoire femelle à tête Allen (51) pour entrer en prise avec la tête mâle sur un outil Allen, faisant ainsi tourner le raccord (50) et changeant la distance entre les premier et second éléments ancrables auto-expansibles (30a, b).

14. Système selon une quelconque des revendications précédentes, dans lequel les premier et second éléments ancrables auto-expansibles (30a, b) sont de formes et/ou tailles différentes.

15. Système selon la revendication 14, dans lequel les premier et second éléments ancrables auto-expansibles (30a, b) sont de taille différente, présentent une expansion radiale différente de la configuration libérée, et/ou sont différents en ce qui concerne la symétrie ou asymétrie.
